# EUROPEAN PATENT APPLICATION

(11) **EP 4 471 132 A1**
(43) Date of publication of application: **04.12.2024**
(21) Application number: 23745906.0
(22) Date of filing: 09.01.2023
(51) Int. Cl.: C12N 7/01, C07K 14/145, A61K 35/766, A61P 35/00, C12R 1/93

(54) **ONCOLYTIC VIRUS AND USE THEREOF**

(30) Priority: 26.01.2022 CN 202210094869
(71) Applicant: Joint Biosciences (SH) Ltd., Pudong New Area Shanghai 201318 (CN)
(72) Inventor: ZHOU, Guoqing, Shanghai 201318 (CN); ZHANG, Fan, Shanghai 201318 (CN); MA, Liang, Shanghai 201318 (CN); TIAN, Ting, Shanghai 201318 (CN)
(74) Representative: Ullrich & Naumann PartG mbB
(86) International application number: PCT/CN2023/071375
(87) International publication number: WO 2023/143023

(57) **Abstract**

The present application discloses an oncolytic virus and a use thereof. The oncolytic virus comprises an M protein, in which the M protein includes amino acid substitution(s) at one or more of the following positions compared to an amino acid sequence set forth in SEQ ID NO 1: position 32, position 33, position 49, position 54, position 133, and position 225. Further disclosed are an expression vector for the oncolytic virus, a virus production cell capable of producing the oncolytic virus, and a pharmaceutical composition including the oncolytic virus, as well as a method for preparing the oncolytic virus, the expression vector for the oncolytic virus, the virus production cell, and/or the pharmaceutical composition, and a use of the oncolytic virus, the expression vector for the oncolytic virus, the virus production cell, and/or the pharmaceutical composition. The present application can effectively improve the safety and cure rate of the oncolytic virus.

## Description

### FIELD OF THE INVENTION

The present application relates to the technical field of biomedicine, and, in particular, to an oncolytic virus and a use thereof.

### BACKGROUND OF THE INVENTION

The Oncolytic viruses are a type of tumor-killing viruses with replication ability, currently widely accepted as an important branch of tumor immunotherapy. The oncolytic viruses can specifically target and infect tumor cells, for example, by utilizing the inactivation or deficiency of tumor suppressor genes in tumor cells to selectively infect tumor cells. After infecting tumor cells, the oncolytic viruses will replicate in large quantities in tumor cells and eventually destroy the tumor cells, thereby killing the tumor cells. Moreover, the oncolytic viruses can also provide immune stimulation signals necessary to enhance the host's own anti-cancer response, thereby attracting more immune cells to continuously kill residual tumor cells.

In spite of a good application prospect of the oncolytic viruses in tumor immunotherapy, wild-type oncolytic viruses often cause problems, for example, leading to inflammation in the nervous system of a body. In the process of using wild-type viruses to infect tumor cells, they also imposes a greater risk of pathogenicity. Therefore, in order to further promote the clinical application of oncolytic viruses, it is necessary to modify the wild-type oncolytic viruses to obtain attenuated oncolytic viruses. The attenuated oncolytic viruses are used in clinical applications to reduce the pathogenic risk of the oncolytic viruses and increase the safety of the oncolytic viruses.

However, for modifying oncolytic viruses, if only the wild-type oncolytic viruses are randomly genetically modified, their cytotoxicity might be reduced, but the modified oncolytic viruses might have a poor cure rate, or even be unable to be packaged, which is not conducive to the promotion of the clinical application of the oncolytic viruses. Therefore, providing a modified oncolytic virus with good safety and high cure rate has important scientific research value and application significance in the field of tumor immunotherapy.

### SUMMARY

In order to increase the safety and cure rate of oncolytic viruses, the present application provides an oncolytic virus and a use thereof.

In a first aspect, the oncolytic virus of the present application adopts the following technical solutions:

an oncolytic virus, including an M protein, wherein the M protein includes an amino acid sequence with amino acid substitution(s) at one or more of the following positions compared to an amino acid sequence set forth in SEQ ID NO 1: position 32, position 33, position 49, position 54, position 133, and position 225.

In some specific embodiments, the amino acid substitution of the M protein includes mutation of asparagine at position 32 to serine (N32S); and/or mutation of methionine at position 33 to alanine (M33A); and/or mutation of asparagine at position 49 to aspartic acid (N49D); and/or mutation of histidine at position 54 to tyrosine (H54Y); and/or mutation of alanine at position 133 to threonine (A133T); and/or mutation of valine at position 225 to isoleucine (V225I).

Further, the M protein further includes an amino acid sequence with amino acid substitution(s) at one or more of the following positions: position 21, position 51, position 111, position 221 and position 226.

In some specific embodiments, the amino acid substitution of the M protein further includes mutation of glycine at position 21 to glutamic acid (G21E); and/or, the amino acid substitution of the M protein includes mutation of methionine at position 51 to arginine (M51R); and/or, mutation of methionine at position 51 to alanine (M51A); and/or, mutation of leucine at position 111 to alanine (L111A); and/or, mutation of valine at position 221 to phenylalanine (V221F); and/or, mutation of serine at position 226 to arginine (S226R).

In a specific embodiment, the amino acid substitution of the M protein includes the mutation of glycine at position 21 to glutamic acid (G21E).

In a specific embodiment, the amino acid substitution of the M protein includes the mutation of asparagine at position 32 to serine (N32S).

In a specific embodiment, the amino acid substitution of the M protein includes the mutation of methionine at position 33 to alanine (M33A).

In a specific embodiment, the amino acid substitution of the M protein includes the mutation of asparagine at position 49 to aspartic acid (N49D).

In a specific embodiment, the amino acid substitution of the M protein includes the mutation of histidine at position 54 to tyrosine (H54Y).

In a specific embodiment, the amino acid substitution of the M protein includes the mutation of leucine at position 111 to alanine (L1 1 1A).

In a specific embodiment, the amino acid substitution of the M protein includes the mutation of alanine at position 133 to threonine (A133T).

In a specific embodiment, the amino acid substitution of the M protein includes the mutation of valine at position 225 to isoleucine (V225I).

In a specific embodiment, the amino acid substitution of the M protein includes the mutation of methionine at position 51 to arginine (M51R).

In a specific embodiment, the amino acid substitution of the M protein includes the mutation of methionine at position 51 to alanine (M51A).

In a specific embodiment, the amino acid substitution of the M protein includes the mutation of valine at position 221 to phenylalanine (V221F).

In a specific embodiment, the amino acid substitution of the M protein includes the mutation of serine at position 226 to arginine (S226R).

In a specific embodiment, the amino acid substitution of the M protein includes mutation of serine at position 226 to glycine (S226G).

In a specific embodiment, the M protein has the amino acid substitution of G21E.

In a specific embodiment, the M protein has the amino acid substitutions of G21E and N32S.

In a specific embodiment, the M protein has the amino acid substitutions of G21E, N32S and M33A.

In a specific embodiment, the M protein has the amino acid substitutions of G21E, N32S, M33A, and N49D.

In a specific embodiment, the M protein has the amino acid substitutions of G21E, N32S, M33A, N49D, and H54Y.

In a specific embodiment, the M protein has the amino acid substitutions of G21E, N32S, M33A, N49D, H54Y, and L111A.

In a specific embodiment, the M protein has the amino acid substitutions of G21E, N32S, M33A, N49D, H54Y, L111A, and A133T.

In a specific embodiment, the M protein has the amino acid substitutions of G21E, N32S, M33A, N49D, H54Y, L111A, A133T, and V225I.

In a specific embodiment, the M protein has the amino acid substitutions of G21E, N32S, M33A, N49D, M51R, H54Y, L111A, A133T, and V225I.

In a specific embodiment, the M protein has the amino acid substitutions of G21E, N32S, M33A, N49D, M51R, H54Y, L111A, A133T, V221F, and V225I.

In a specific embodiment, the M protein has the amino acid substitutions of G21E, N32S, M33A, N49D, M51R, H54Y, L111A, A133T, V221F, V225I, and S226R.

In a specific embodiment, the M protein has the amino acid substitutions of N32S, M33A, N49D, M51R, H54Y, L111A, A133T, V221F, V225I, and S226R.

In a specific embodiment, the M protein has the amino acid substitutions of M33A, N49D, M51R, H54Y, L111A, A133T, V221F, V225I, and S226R.

In a specific embodiment, the M protein has the amino acid substitutions of N49D, M51R, H54Y, L111A, A133T, V221F, V225I, and S226R.

In a specific embodiment, the M protein has the amino acid substitutions of M51R, H54Y, L111A, A133T, V221F, V225I, and S226R.

In a specific embodiment, the M protein has the amino acid substitutions of H54Y, L111A, A133T, V221F, V225I, and S226R.

In a specific embodiment, the M protein has the amino acid substitutions of L111A, A133T, V221F, V225I, and S226R.

In a specific embodiment, the M protein has the amino acid substitutions of A133T, V221F, V225I, and S226R.

In a specific embodiment, the M protein has the amino acid substitutions of V221F, V225I, and S226R.

In a specific embodiment, the M protein has the amino acid substitutions of V225I and S226R.

In a specific embodiment, the M protein has the amino acid substitution of S226R.

In a specific embodiment, the M protein has the amino acid substitutions of N32S, N49D, H54Y, and V225I.

In a specific embodiment, the M protein has the amino acid substitutions of N32S, N49D, H54Y, V225I, and S226G

In a specific embodiment, the M protein has the amino acid substitutions of N32S, N49D, M51R, H54Y, V221F, V225I, and S226R.

In a specific embodiment, the M protein has the amino acid substitutions of N32S, M33A, N49D, M51R, H54Y, V221F, V225I, and S226R.

In a specific embodiment, the M protein has the amino acid substitutions of N32S, N49D, M51R, H54Y, A133T, V221F, V225I, and S226R.

In a specific embodiment, the M protein has the amino acid substitutions of N32S, M33A, N49D, M51R, H54Y, A133T, V221F, V225I, and S226R.

In a specific embodiment, the M protein has the amino acid substitutions of G21E, N32S, N49D, M51A, H54Y, L111A, V225I, and S226R.

In some embodiments, the M protein includes an amino acid sequence as set forth in SEQ ID NO 2.

In some embodiments, the M protein includes an amino acid sequence as set forth in SEQ ID NO 3.

In some embodiments, the M protein includes an amino acid sequence as set forth in SEQ ID NO 4.

In some embodiments, the M protein includes an amino acid sequence as set forth in SEQ ID NO 5.

In some embodiments, the M protein includes an amino acid sequence as set forth in SEQ ID NO 6.

In some embodiments, the M protein includes an amino acid sequence as set forth in SEQ ID NO 7.

In some embodiments, the M protein includes an amino acid sequence as set forth in SEQ ID NO 8.

In some embodiments, the M protein includes an amino acid sequence as set forth in SEQ ID NO 9.

In some embodiments, the M protein includes an amino acid sequence as set forth in SEQ ID NO 10.

In some embodiments, the M protein includes an amino acid sequence as set forth in SEQ ID NO 11.

In some embodiments, the M protein includes an amino acid sequence as set forth in SEQ ID NO 12.

In some embodiments, the M protein includes an amino acid sequence as set forth in SEQ ID NO 13.

In some embodiments, the M protein includes an amino acid sequence as set forth in SEQ ID NO 14.

In some embodiments, the M protein includes an amino acid sequence as set forth in SEQ ID NO 15.

In some embodiments, the M protein includes an amino acid sequence as set forth in SEQ ID NO 16.

In some embodiments, the M protein includes an amino acid sequence as set forth in SEQ ID NO 17.

In some embodiments, the M protein includes an amino acid sequence as set forth in SEQ ID NO 18.

In some embodiments, the M protein includes an amino acid sequence as set forth in SEQ ID NO 19.

In some embodiments, the M protein includes an amino acid sequence as set forth in SEQ ID NO 20.

In some embodiments, the M protein includes an amino acid sequence as set forth in SEQ ID NO 21.

In some embodiments, the M protein includes an amino acid sequence as set forth in SEQ ID NO 22.

In some embodiments, the M protein includes an amino acid sequence as set forth in SEQ ID NO 23.

In some embodiments, the M protein includes an amino acid sequence as set forth in SEQ ID NO 24.

In some embodiments, the M protein includes an amino acid sequence as set forth in SEQ ID NO 25.

In some embodiments, the M protein includes an amino acid sequence as set forth in SEQ ID NO 26.

In some embodiments, the M protein includes an amino acid sequence as set forth in SEQ ID NO 27.

In some embodiments, the M protein includes an amino acid sequence as set forth in SEQ ID NO 28.

In some embodiments, the M protein includes an amino acid sequence as set forth in SEQ ID NO 29.

In some embodiments, the M protein includes an amino acid sequence as set forth in SEQ ID NO 30.

An oncolytic virus, including the described M protein and further including a G protein, wherein the G protein includes an amino acid sequence with amino acid substitution(s) at one or more of the following positions compared to an amino acid sequence set forth in SEQ ID NO 31: position 438, position 453, position 471, and position 487.

In some specific embodiments, the G protein further includes an amino acid sequence with amino acid substitution(s) at one or more of the following positions: position 53, position 141, position 172, position 217, position 232, position 331, position 371 and position 436.

In some specific embodiments, the amino acid substitution of the G protein includes mutation of valine at position 53 to isoleucine (V53I); and/or mutation of alanine at position 141 to valine (A141V); and/or mutation of aspartic acid at position 172 to tyrosine (D172Y); and/or mutation of lysine at position 217 to glutamic acid (K217E); and/or mutation of aspartic acid at position 232 to glycine (D232G); and/or mutation of valine at position 331 to alanine (V331A); and/or, mutation of valine at position 371 to glutamic acid (V371E); and/or, mutation of glycine at position 436 to aspartic acid (G436D); and/or, mutation of threonine at position 438 to serine (T438S); and/or, mutation of phenylalanine at position 453 to leucine (F453L); and/or, mutation of threonine at position 471 to isoleucine (T471I); and/or, mutation of tyrosine at position 487 to histidine (Y487H).

In a specific embodiment, the amino acid substitution of the G protein includes mutation of valineat position 53 to isoleucine (V53I).

In a specific embodiment, the amino acid substitution of the G protein includes mutation of alanine at position 141 to valine (A141V).

In a specific embodiment, the amino acid substitution of the G protein includes mutation of aspartic acid at position 172 to tyrosine (D172Y).

In a specific embodiment, the amino acid substitution of the G protein includes mutation of lysine at position 217 to glutamic acid (K217E).

In a specific embodiment, the amino acid substitution of the G protein includes mutation of aspartic acid at position 232 to glycine (D232G).

In a specific embodiment, the amino acid substitution of the G protein includes mutation of valine at position 331 to alanine (V331A).

In a specific embodiment, the amino acid substitution of the G protein includes mutation of valine at position 371 to glutamic acid (V371E).

In a specific embodiment, the amino acid substitution of the G protein includes mutation of glycine at position 436 to aspartic acid (G436D).

In a specific embodiment, the amino acid substitution of the G protein includes mutation of threonine at position 438 to serine (T438S).

In a specific embodiment, the amino acid substitution of the G protein includes mutation of phenylalanine at position 453 to leucine (F453L).

In a specific embodiment, the amino acid substitution of the G protein includes mutation of threonine at position 471 to isoleucine (T471I).

In a specific embodiment, the amino acid substitution of the G protein includes mutation of tyrosine at position 487 to histidine (Y487H).

In a specific embodiment, the G protein has the amino acid substitution of V53I.

In a specific embodiment, the G protein has the amino acid substitutions of V53I and A141V.

In a specific embodiment, the G protein has the amino acid substitutions of V53I, A141V and D172Y.

In a specific embodiment, the G protein has the amino acid substitutions of V53I, A141V, D172Y, and K217E.

In a specific embodiment, the G protein has the amino acid substitutions of V53I, A141V, D172Y, K217E, and D232G

In a specific embodiment, the G protein has the amino acid substitutions of V53I, A141V, D172Y, K217E, D232G, and V331A.

In a specific embodiment, the G protein has the amino acid substitutions of V53I, A141V, D172Y, K217E, D232G, V331A, and V371E.

In a specific embodiment, the G protein has the amino acid substitutions of V53I, A141V, D172Y, K217E, D232G, V331A, V371E, and G436D.

In a specific embodiment, the G protein has the amino acid substitutions of V53I, A141V, D172Y, K217E, D232G, V331A, V371E, G436D, and T438S.

In a specific embodiment, the G protein has the amino acid substitutions of V53I, A141V, D172Y, K217E, D232G, V331A, V371E, G436D, T438S, and F453L.

In a specific embodiment, the G protein has the amino acid substitutions of V53I, A141V, D172Y, K217E, D232G, V331A, V371E, G436D, T438S, F453L, and T471I.

In a specific embodiment, the G protein has the amino acid substitutions of V53I, A141V, D172Y, K217E, D232G, V331A, V371E, G436D, T438S, F453L, T471I, and Y487H.

In a specific embodiment, the G protein has the amino acid substitutions of A141V, D172Y, K217E, D232G, V331A, V371E, G436D, T438S, F453L, T471I, and Y487H.

In a specific embodiment, the G protein has the amino acid substitutions of D172Y, K217E, D232G, V331A, V371E, G436D, T438S, F453L, T471I, and Y487H.

In a specific embodiment, the G protein has the amino acid substitutions of K217E, D232G, V331A, V371E, G436D, T438S, F453L, T471I, and Y487H.

In a specific embodiment, the G protein has the amino acid substitutions of D232G, V331A, V371E, G436D, T438S, F453L, T471I, and Y487H.

In a specific embodiment, the G protein has the amino acid substitutions of V331A, V371E, G436D, T438S, F453L, T471I, and Y487H.

In a specific embodiment, the G protein has the amino acid substitutions of V371E, G436D, T438S, F453L, T471I, and Y487H.

In a specific embodiment, the G protein has the amino acid substitutions of G436D, T438S, F453L, T471I, and Y487H.

In a specific embodiment, the G protein has the amino acid substitutions of T438S, F453L, T471I, and Y487H.

In a specific embodiment, the G protein has the amino acid substitutions of F453L, T471I, and Y487H.

In a specific embodiment, the G protein has the amino acid substitutions of T471I and Y487H.

In a specific embodiment, the G protein has the amino acid substitution of Y487H.

In some embodiments, the G protein includes an amino acid sequence as set forth in SEQ ID NO 32.

In some embodiments, the G protein includes an amino acid sequence as set forth in SEQ ID NO 33.

In some embodiments, the G protein includes an amino acid sequence as set forth in SEQ ID NO 34.

In some embodiments, the G protein includes an amino acid sequence as set forth in SEQ ID NO 35.

In some embodiments, the G protein includes an amino acid sequence as set forth in SEQ ID NO 36.

In some embodiments, the G protein includes an amino acid sequence as set forth in SEQ ID NO 37.

In some embodiments, the G protein includes an amino acid sequence as set forth in SEQ ID NO 38.

In some embodiments, the G protein includes an amino acid sequence as set forth in SEQ ID NO 39.

In some embodiments, the G protein includes an amino acid sequence as set forth in SEQ ID NO 40.

In some embodiments, the G protein includes an amino acid sequence as set forth in SEQ ID NO 41.

In some embodiments, the G protein includes an amino acid sequence as set forth in SEQ ID NO 42.

In some embodiments, the G protein includes an amino acid sequence as set forth in SEQ ID NO 43.

In some embodiments, the G protein includes an amino acid sequence as set forth in SEQ ID NO 44.

In some embodiments, the G protein includes an amino acid sequence as set forth in SEQ ID NO 45.

In some embodiments, the G protein includes an amino acid sequence as set forth in SEQ ID NO 46.

In some embodiments, the G protein includes an amino acid sequence as set forth in SEQ ID NO 47.

In some embodiments, the G protein includes an amino acid sequence as set forth in SEQ ID NO 48.

In some embodiments, the G protein includes an amino acid sequence as set forth in SEQ ID NO 49.

In some embodiments, the G protein includes an amino acid sequence as set forth in SEQ ID NO 50.

In some embodiments, the G protein includes an amino acid sequence as set forth in SEQ ID NO 51.

In some embodiments, the G protein includes an amino acid sequence as set forth in SEQ ID NO 52.

In some embodiments, the G protein includes an amino acid sequence as set forth in SEQ ID NO 53.

In some embodiments, the G protein includes an amino acid sequence as set forth in SEQ ID NO 54.

An oncolytic virus, including the described M protein or the described M protein and G protein, and further including an N protein, wherein the N protein includes an amino acid sequence with amino acid substitution(s) at one or more of the following positions compared to an amino acid sequence set forth in SEQ ID NO 55: position 14, position 155, and position 353.

In some specific embodiments, the amino acid substitution of the N protein includes mutation of isoleucine at position 14 to valine (I14V); and/or mutation of arginine at position 155 to lysine (R155K); and/or mutation of serine at position 353 to asparagine (S353N).

In a specific embodiment, the amino acid substitution of the N protein includes the mutation of isoleucine at position 14 to valine (I14V).

In a specific embodiment, the amino acid substitution of the N protein includes mutation of arginine at position 155 to lysine (R155K).

In a specific embodiment, the amino acid substitution of the N protein includes mutation of serine at position 353 to asparagine (S353N).

In a specific embodiment, the N protein has the amino acid substitution of I14V.

In a specific embodiment, the N protein has the amino acid substitutions of I14V and R155K.

In a specific embodiment, the N protein has the amino acid substitutions of I14V, R155K, and S353N.

In a specific embodiment, the N protein has the amino acid substitutions of R155K and S353N.

In a specific embodiment, the N protein has the amino acid substitution of S353N.

In some embodiments, the N protein includes an amino acid sequence as set forth in SEQ ID NO 56.

In some embodiments, the N protein includes an amino acid sequence as set forth in SEQ ID NO 57.

In some embodiments, the N protein includes an amino acid sequence as set forth in SEQ ID NO 58.

In some embodiments, the N protein includes an amino acid sequence as set forth in SEQ ID NO 59.

In some embodiments, the N protein includes an amino acid sequence as set forth in SEQ ID NO 60.

An oncolytic virus, including the described M protein, or the described M protein and G protein, or the described M protein, G protein and N protein, or the described M protein and N protein, and further including a P protein, wherein the P protein includes an amino acid sequence with amino acid substitution(s) at one or more of the following positions compared to an amino acid sequence set forth in SEQ ID NO 61: position 50, position 76, position 99, position 126, position 140, position 151, position 168, position 170, position 189, and position 237.

In some specific embodiments, the amino acid substitution of the P protein includes mutation of arginine at position 50 to lysine (R50K); and/or mutation of valine at position 76 to alanine (V76A); and/or mutation of asparagine at position 99 to glutamic acid (D99E); and/or mutation of leucine at position 126 to serine (L126S); and/or mutation of leucine at position 140 to serine (L140S); and/or mutation of histidine at position 151 to tyrosine (H151Y); and/or mutation of isoleucine at position 168 to methionine (I168M); and/or mutation of lysine at position 170 to glutamic acid (K170E); and/or mutation of tyrosine at position 189 to serine (Y189S); and/or mutation of asparagine at position 237 to aspartic acid (N237D).

In a specific embodiment, the amino acid substitution of the P protein includes mutation of arginine at position 50 to lysine (R50K).

In a specific embodiment, the amino acid substitution of the P protein includes mutation of valine at position 76 to alanine (V76A).

In a specific embodiment, the amino acid substitution of the P protein includes the mutation of asparagine at position 99 to glutamic acid (D99E).

In a specific embodiment, the amino acid substitution of the P protein includes mutation of leucine at position 126 to serine (L126S).

In a specific embodiment, the amino acid substitution of the P protein includes mutation of leucine at position 140 to serine (L140S).

In a specific embodiment, the amino acid substitution of the P protein includes mutation of histidine at position 151 to tyrosine (H151Y).

In a specific embodiment, the amino acid substitution of the P protein includes mutation of isoleucine at position 168 to methionine (I168M).

In a specific embodiment, the amino acid substitution of the P protein includes mutation of lysine at position 170 to glutamic acid (K170E).

In a specific embodiment, the amino acid substitution of the P protein includes mutation of tyrosine at position 189 to serine (Y189S).

In a specific embodiment, the amino acid substitution of the P protein includes mutation of asparagine at position 237 to aspartic acid (N237D).

In a specific embodiment, the P protein has the amino acid substitution of R50K.

In a specific embodiment, the P protein has the amino acid substitutions of R50K and V76A.

In a specific embodiment, the P protein has the amino acid substitution of R50K, V76A, and D99E.

In a specific embodiment, the P protein has the amino acid substitutions of R50K, V76A, D99E, and L126S.

In a specific embodiment, the P protein has the amino acid substitutions of R50K, V76A, D99E, L126S, and L140S.

In a specific embodiment, the P protein has the amino acid substitutions of R50K, V76A, D99E, L126S, L140S, and H151Y

In a specific embodiment, the P protein has the amino acid substitutions of R50K, V76A, D99E, L126S, L140S, H151Y, and I168M.

In a specific embodiment, the P protein has the amino acid substitutions of R50K, V76A, D99E, L126S, L140S, H151Y, I168M, and K170E.

In a specific embodiment, the P protein has the amino acid substitutions of R50K, V76A, D99E, L126S, L140S, H151Y, I168M, K170E, and Y189S.

In a specific embodiment, the P protein has the amino acid substitutions of R50K, V76A, D99E, L126S, L140S, H151Y, I168M, K170E, Y189S, and N237D.

In a specific embodiment, the P protein has the amino acid substitutions of V76A, D99E, L126S, L140S, H151Y, I168M, K170E, Y189S, and N237D.

In a specific embodiment, the P protein has the amino acid substitutions of D99E, L126S, L140S, H151Y, I168M, K170E, Y189S, and N237D.

In a specific embodiment, the P protein has the amino acid substitutions of L126S, L140S, H151Y, I168M, K170E, Y189S, and N237D.

In a specific embodiment, the P protein has the amino acid substitutions of L140S, H151Y, I168M, K170E, Y189S, and N237D.

In a specific embodiment, the P protein has the amino acid substitutions of H151Y, I168M, K170E, Y189S, and N237D.

In a specific embodiment, the P protein has the amino acid substitutions of I168M, K170E, Y189S, and N237D.

In a specific embodiment, the P protein has the amino acid substitutions of K170E, Y189S, and N237D.

In a specific embodiment, the P protein has the amino acid substitutions of Y189S and N237D.

In a specific embodiment, the P protein has the amino acid substitution of N237D.

In some embodiments, the P protein includes an amino acid sequence as set forth in SEQ ID NO 62.

In some embodiments, the P protein includes an amino acid sequence as set forth in SEQ ID NO 63.

In some embodiments, the P protein includes an amino acid sequence as set forth in SEQ ID NO 64.

In some embodiments, the P protein includes an amino acid sequence as set forth in SEQ ID NO 65.

In some embodiments, the P protein includes an amino acid sequence as set forth in SEQ ID NO 66.

In some embodiments, the P protein includes an amino acid sequence as set forth in SEQ ID NO 67.

In some embodiments, the P protein includes an amino acid sequence as set forth in SEQ ID NO 68.

In some embodiments, the P protein includes an amino acid sequence as set forth in SEQ ID NO 69.

In some embodiments, the P protein includes an amino acid sequence as set forth in SEQ ID NO 70.

In some embodiments, the P protein includes an amino acid sequence as set forth in SEQ ID NO 71.

In some embodiments, the P protein includes an amino acid sequence as set forth in SEQ ID NO 72.

In some embodiments, the P protein includes an amino acid sequence as set forth in SEQ ID NO 73.

In some embodiments, the P protein includes an amino acid sequence as set forth in SEQ ID NO 74.

In some embodiments, the P protein includes an amino acid sequence as set forth in SEQ ID NO 75.

In some embodiments, the P protein includes an amino acid sequence as set forth in SEQ ID NO 76.

In some embodiments, the P protein includes an amino acid sequence as set forth in SEQ ID NO 77.

In some embodiments, the P protein includes an amino acid sequence as set forth in SEQ ID NO 78.

In some embodiments, the P protein includes an amino acid sequence as set forth in SEQ ID NO 79.

In some embodiments, the P protein includes an amino acid sequence as set forth in SEQ ID NO 80.

An oncolytic virus, including the described M protein, or the described M protein and G protein, or the described M protein, G protein and N protein, or the described M protein, G protein, N protein and P protein, or the described M protein and N protein, or the described M protein and P protein, or the described M protein, G protein and P protein, or the described M protein, N protein and P protein, and further including an L protein, wherein the L protein includes an amino acid sequence with amino acid substitution(s) at one or more of the following positions compared to an amino acid sequence set forth in SEQ ID NO 81: position 87 and position 487.

In some specific embodiments, the amino acid substitution of the L protein includes mutation of serine at position 87 to proline (S87P); and/or mutation of isoleucine at position 487 to threonine (I487T).

In a specific embodiment, the amino acid substitution of the L protein includes the mutation of serine at position 87 to proline (S87P).

In a specific embodiment, the amino acid substitution of the L protein includes the mutation of isoleucine at position 487 to threonine (I487T).

In a specific embodiment, the L protein has the amino acid substitution of S87P.

In a specific embodiment, the L protein has the amino acid substitutions of S87P and I487T.

In a specific embodiment, the L protein has the amino acid substitution of I487T.

In some embodiments, the L protein includes an amino acid sequence as set forth in SEQ ID NO 82.

In some embodiments, the L protein includes an amino acid sequence as set forth in SEQ ID NO 83.

In some embodiments, the L protein includes an amino acid sequence as set forth in SEQ ID NO 84.

In some specific embodiments, the oncolytic virus is obtained by site-directed mutagenesis based on a rod-shaped virus.

In some specific embodiments, the oncolytic virus is obtained by site-directed mutagenesis based on a vesicular stomatitis virus (VSV).

In some specific embodiments, the oncolytic virus is obtained by site-directed mutagenesis based on an Indiana MuddSummer subtype strain of the VSV

In some specific embodiments, the oncolytic virus includes or expresses an foreign target protein.

In some specific embodiments, the oncolytic virus includes a nucleic acid molecule, which includes a nucleic acid sequence encoding the M protein with the amino acid substitution(s), and/or a nucleic acid sequence encoding the G protein with the amino acid substitution(s), and/or a nucleic acid sequence encoding the N protein with the amino acid substitution(s), and/or a nucleic acid sequence encoding the P protein with the amino acid substitution(s), and/or a nucleic acid sequence encoding the L protein with the amino acid substitution(s).

In some specific embodiments, the nucleic acid molecule includes a nucleic acid sequence encoding the foreign target protein.

In some specific embodiments, in the nucleic acid molecule, the nucleic acid sequence encoding the foreign target protein is positioned between the nucleic acid sequence encoding the M protein with the amino acid substitution(s), and/or the nucleic acid sequence encoding the G protein with the amino acid substitution(s), and/or the nucleic acid sequence encoding the N protein with the amino acid substitution(s), and/or the nucleic acid sequence encoding the P protein with the amino acid substitution(s), and/or the nucleic acid sequence encoding the L protein with the amino acid substitution(s).

In a second aspect, the present application provides an oncolytic virus expression vector, adopting the following technical solution:

an oncolytic virus expression vector, capable of producing any of the oncolytic viruses described in the present application.

In a third aspect, the present application provides a virus production cell, adopting the following technical solution:

a virus production cell, capable of producing any oncolytic virus described in the present application.

In a fourth aspect, the present application provides a pharmaceutical composition, adopting the following technical solution:

a pharmaceutical composition, including any of the oncolytic viruses described in the present application and, optionally, a pharmaceutically acceptable carrier.

In a fifth aspect, the present application provides a method for preparing the described oncolytic virus, the described oncolytic virus expression vector, the described virus production cell and/or the described pharmaceutical composition.

In a sixth aspect, the present application provides a use of the described oncolytic virus, the described oncolytic virus expression vector, the described virus production cell and/or the described pharmaceutical composition in the preparation of a drug for prevention and/or treatment of a disease and/or disorder.

In some specific embodiments, the described oncolytic virus, the described oncolytic virus expression vector, the described virus production cell and/or the described pharmaceutical composition are/is used in a method for killing an abnormally proliferative cell in a slow and sustained manner.

In some specific embodiments, the disease and/or disorder includes: an abnormally proliferative cell, selected from a tumor cell and a cell related to tumor tissues; preferably, the tumor cell is a cancer cell; more preferably, the cancer cell is a metastatic cancer cell.

In some specific embodiments, the tumor includes a solid tumor and/or a hematological tumor.

In summary, the present application has the following beneficial effects.

The oncolytic viruses of the present application have good infection ability and *in vitro* cytotoxicity to abnormally proliferative (tumor) LLC cell, 4T1 cell line, MC38 cell and Hela cell, and are not easily cleared from the LLC cell, the 4T1 cell, the MC38 cell and the Hela cell. In addition, the oncolytic viruses of the present application have poor infection ability and in vitro cytotoxicity to a normal MEF cell and are all easily cleared from the normal MEF cell. Therefore, the oncolytic viruses of the present application can be well used for the infection and killing of tumors, cancers and other cells, and are not easily cleared from tumors, cancers and other cells, further improving the cure rate of oncolytic viruses for tumors, cancers and other cells. Meanwhile, the oncolytic viruses described above will not cause damages to normal cells and can be more easily cleared from normal cells, thereby further ensuring the safety of normal cells.

### BRIEF DESCRIPTION OF THE DRAWINGS

FIG. 1 shows test results of the oncolytic viruses of the present application and the wild-type oncolytic virus in infection ability to the LLC cell.
FIG. 2 shows test results of the oncolytic viruses of the present application and the wild-type oncolytic virus in infection ability to the 4T1 cell.
FIG. 3 shows test results of the oncolytic viruses of the present application and the wild-type oncolytic virus in infection ability to the MC38 cell.
FIG. 4 shows test results of the oncolytic viruses of the present application and the wild-type oncolytic virus in infection ability to the Hela cell.
FIG. 5 shows test results of the oncolytic viruses of the present application and the wild-type oncolytic virus in infection ability to the MEF cell.
FIG. 6 shows test results of the oncolytic viruses of the present application and the wild-type oncolytic virus in in vitro cytotoxicity against the LLC cell.
FIG. 7 shows test results of the oncolytic viruses of the present application and the wild-type oncolytic virus in in vitro cytotoxicity against the 4T1 cell.
FIG. 8 shows test results of the oncolytic viruses of the present application and the wild-type oncolytic virus in in vitro cytotoxicity against the MC38 cell.
FIG. 9 shows test results of the oncolytic viruses of the present application and the wild-type oncolytic virus in in vitro cytotoxicity against the Hela cell.
FIG. 10 shows test results of the oncolytic viruses of the present application and the wild-type oncolytic virus in in vitro cytotoxicity against the MEF cell.
FIG. 11 shows the expression of IFN-β induced by the oncolytic viruses of the present application and the wild-type oncolytic virus in the LLC cell.
FIG. 12 shows the expression of IFN-β induced by the oncolytic viruses of the present application and the wild-type oncolytic virus in the 4T1 cell.
FIG. 13 shows the expression of IFN-β induced by the oncolytic viruses of the present application and the wild-type oncolytic virus in the MC38 cell.
FIG. 14 shows the expression of IFN-β induced by the oncolytic viruses of the present application and the wild-type oncolytic virus in the Hela cell.
FIG. 15 shows the expression of IFN-β induced by the oncolytic virus of the present application and the wild-type oncolytic virus in the MEF cell.

In the above figures, the number 0 on the horizontal axis represents the wild-type oncolytic virus; the numbers 1-79 on the horizontal axis represent the oncolytic viruses prepared in Preparation Examples 1-79, respectively.

Log₁₀ TCID50 on the vertical axis represents the values of TCID50 calculated by a Karber method. The larger the value of Log₁₀ TCID50, the better the infection ability of an oncolytic virus to the cell; the smaller the value of Log₁₀ TCID50, the worse the infection ability of an oncolytic virus to the cell;

OD₅₇₀ on the vertical axis represents the OD value of the cell. The larger the value of OD₅₇₀, the worse the cytotoxicity of an oncolytic virus against the cell; the smaller the value of OD₅₇₀, the better the cytotoxicity of an oncolytic virus against the cell.

The IFN-β level on the vertical axis represents the expression level of the IFN-β gene. The larger the value of the IFN-β level, the weaker the reproduction ability of an oncolytic virus in the cell and the easier it is to clear the oncolytic virus; the smaller the value of the IFN-β level, the stronger the reproduction ability of the oncolytic virus in the cell, and the more difficult it is to clear the oncolytic virus.

Other aspects and advantages of the present application can be readily perceived by those skilled in the art from the detailed description below. The detailed description below only shows and describes the exemplary embodiments of the present application. As would be appreciated by those skilled in the art, the content of the present application allows those skilled in the art to change the specific embodiments disclosed without departing from the spirit and scope of the present application. Accordingly, the accompanying drawings and the description of the present application are merely illustrative and not restrictive.

### DETAILED DESCRIPTION

The implementation of the invention of this application will be described below with specific embodiments. Those skilled in the art can easily understand other advantages and effects of the invention of this application from the contents disclosed herein.

### DEFINITIONS

The term "oncolytic virus", as used herein, generally refers to a virus that can replicate in tumor cells and kill tumor cells. The oncolytic virus includes, but is not limited to, vesicular stomatitis virus (referred to as "VSV"), poxvirus, herpes simplex virus, measles virus, Semliki Forest virus, poliovirus, reovirus, Seneca Valley virus, Echo-type enterovirus, coxsackievirus, Newcastle disease virus and Maraba virus. In certain embodiments, the oncolytic virus is modified to improve the selectivity for tumor cells. In certain embodiments, the oncolytic virus is modified to reduce its immunogenicity. In some embodiments, the oncolytic virus described in the present application is the VSV In some embodiments, the VSV is a mutant of the Indiana MuddSummer subtype strain of the VSV In some embodiments, the M protein, and/or the G protein, and/or the N protein, and/or the P protein, and/or the L protein of the VSV may be subjected to site-directed mutagenesis.

In certain embodiments, the oncolytic virus described in the present application may be an oncolytic virus modified at a gene level, such as modified with one or more genes, so as to improve its tumor selectivity and/or preferentially replicate in dividing cells. The modification at the gene level may be a modification of genes involved in DNA replication, nucleic acid metabolism, host tropism, surface attachment, virulence, lysis and diffusion processes, or may be a modification of integrated foreign genes. The foreign genes may include foreign immunomodulatory genes, foreign screening genes, and foreign reporter genes. The modified oncolytic virus may also be an oncolytic virus modified at an amino acid level, such as, by insertion, deletion, or substitution of one or more amino acids.

The term "M protein", as used herein, generally refers to a matrix protein of the VSV The M protein is an important virulence factor of the VSV and a protein in the VSV that is known to interfere with the natural immune response of mice. The term "M protein" also includes its homologs, orthologs, variants, functionally active fragments, etc. In the present application, the M protein in the Indiana MuddSummer subtype strain of a wild-type VSV may include an amino acid sequence as set forth in SEQ ID NO 1. In the present application, the M protein of the oncolytic virus may include amino acid sequences as set forth in SEQ ID NOs 2-29.

The term "G protein", as used herein, generally refers to a glycoprotein of the VSV, also known as the envelope protein. The term "G protein" also includes its homologs, orthologs, variants, functionally active fragments, etc. In the present application, the G protein in the Indiana MuddSummer subtype strain of a wild-type VSV may include an amino acid sequence as set forth in SEQ ID NO 31. In the present application, the G protein of the oncolytic virus may include amino acid sequences as set forth in SEQ ID NOs 32-54.

The term "N protein", as used herein, generally refers to a nucleocapsid protein of the VSV The term "N protein" also includes its homologs, orthologs, variants, functionally active fragments, etc. In the present application, the N protein in the Indiana MuddSummer subtype strain of a wild-type VSV may include an amino acid sequence as set forth in SEQ ID NO 55. In the present application, the N protein of the oncolytic virus may include amino acid sequences as set forth in SEQ ID NOs 56-60.

The term "P protein", as used herein, generally refers to a phosphoprotein of the VSV. The term "P protein" also includes its homologs, orthologs, variants, functionally active fragments, etc. In the present application, the P protein in the Indiana MuddSummer subtype strain of a wild-type VSV may include an amino acid sequence as set forth in SEQ ID NO 61. In the present application, the P protein of the oncolytic virus may include amino acid sequences as set forth in SEQ ID NOs 62-80.

The term "L protein", as used herein, generally refers to an RNA poly E protein of the VSV. An L gene of the VSV encodes the RNA poly E protein. The term "L protein" also includes its homologs, orthologs, variants, functionally active fragments, etc. In the present application, the L protein in the Indiana MuddSummer subtype strain of a wild-type VSV may include an amino acid sequence as set forth in SEQ ID NO 81. In the present application, the L protein of the oncolytic virus may include amino acid sequences as set forth in SEQ ID NOs 82-84.

In the present application, a protein mutation site is generally expressed by "amino acid + amino acid position + (amino acid after mutation)". In the present application, the mutation may include but is not limited to the addition, replacement, and/or deletion of an amino acid. For example, the term "M51R" generally refers to the mutation of methionine M at position 51 to arginine R.

The term "amino acid substitution", as used herein, generally refers to the replacement of an amino acid residue present in a parent sequence with another amino acid residue. The amino acid in the parent sequence may be replaced, for example, via chemical peptide synthesis or by recombinant methods known in the art. Therefore, "substitution at position xx" generally refers to the replacement of an amino acid present at position xx with an alternative amino acid residue. In the present application, the amino acid substitution may include an amino acid mutation.

In the present application, the term "mutation" generally refers to changing the nucleotide or amino acid sequence of a wild-type molecule. Amino acid changes may include the replacement, deletion, insertion, addition, and truncation of an amino acid, or the processing or cutting of a protein.

The term "nucleic acid molecule", as used herein, generally refers to a nucleotide of any length. The term "nucleic acid molecule", as used herein, may encode a protein contained in the oncolytic virus. In the present application, the nucleic acid molecule may include DNA and/or RNA. In some cases, the RNA may include single-stranded RNA (ssRNA) or double-stranded RNA (dsRNA), and the single-stranded RNA may include sense RNA, antisense RNA, or ambisense RNA.

The term "expression vector", as used herein, generally refers to a nucleic acid vector. Under appropriate conditions, it is generally capable of expressing a target gene and/or a target protein. In certain embodiments of the present application, the expression vector includes a nucleic acid molecule for expressing one or more components of a virus (e.g., an oncolytic virus). For example, the expression vector may include at least one viral genome element and may be packaged into a virus or packaged as a viral particle.

The term "virus-producing cell", as used herein, generally refers to a cell, cell line or cell culture that may or already contains a nucleic acid molecule or expression vector described in the present application, or is capable of expressing the oncolytic virus described in the present application. The cell may include a progeny of a single host cell. The cell may be obtained by in vitro transfection using the expression vector described in the present application.

The term "pharmaceutical composition", as used herein, generally refers to a formulation that is in a form that permits the biological activity of the active ingredient to be effective, and that contains no additional ingredients that are unacceptably toxic to a subject to which the formulation is to be administered. In certain embodiments, these formulations may include active ingredients of a drug and a pharmaceutically acceptable carrier. In certain embodiments, the drug product includes a drug product that is administered by a parenteral, transdermal, intracavitary, intraarterial, intrathecal, and/or intranasal route, or injected directly into tissues. The drug product may be administered by various routes, such as an intravenous, intraperitoneal, subcutaneous, intramuscular, topical or intradermal route.

The term "prevention", as used herein, generally refers to preventing the occurrence and onset, recurrence, and/or spread of a disease or one or more symptoms of the disease by taking certain measures in advance. The term "treatment", as used herein, generally refers to eliminating or improving a disease, or one or more symptoms associated with the disease. In certain embodiments, the treatment generally refers to administering to a patient suffering from the disease one or more drugs to eliminate or alleviate the disease. In certain embodiments, "treatment" may be the administration of the pharmaceutical composition and/or drug product in the presence or absence of other drugs after the onset of symptoms of a particular disease. For example, it may be the use of the pharmaceutical composition and/or drug product described in the present application to prevent the occurrence, development, recurrence and/or metastasis of a tumor.

The term "tumor", as used herein, generally refers to any new pathological growth of tissue. The tumor may be benign or malignant. In the present application, the tumor may be a solid tumor or a hematological tumor. For research use, these tissues may be isolated from readily available sources by methods well known to those skilled in the art.

### DETAILED DESCRIPTION

The present application provides an oncolytic virus, which is based on a wild-type VSV virus, specifically on an Indiana strain of the VSV, the Indiana MuddSummer subtype strain of the VSV, and is obtained by mutating sites on the amino acid sequences of the M protein, G protein, N protein, P protein, and L protein of the Indiana MuddSummer subtype strain of the VSV. The amino acid sequence of the M protein is as set forth in SEQ ID NO 1; the amino acid sequence of the G protein is as set forth in SEQ ID NO 31; the amino acid sequence of the N protein is as set forth in SEQ ID NO 55; the amino acid sequence of the P protein is as set forth in SEQ ID NO 61; and the amino acid sequence of the L protein is as set forth in SEQ ID NO 81. In the present application, the M protein, the G protein, the N protein, the P protein, and the L protein can all be modified.

The present application modifies the VSV as follows to obtain an oncolytic virus.

An oncolytic virus includes an M protein, wherein the M protein includes amino acid substitution(s) at one or more of the following positions compared to the amino acid sequence set forth in SEQ ID NO 1: position 32, position 33, position 49, position 54, position 133, and position 225. The M protein further includes amino acid substitution(s) at one or more of the following positions: position 21, position 51, position 111, position 221, and position 226.

The amino acid substitution of the M protein includes mutation of asparagine at position 32 to serine (N32S); and/or mutation of methionine at position 33 to alanine (M33A); and/or mutation of asparagine at position 49 to aspartic acid (N49D); and/or mutation of histidine at position 54 to tyrosine (H54Y); and/or mutation of alanine at position 133 to threonine (A133T); and/or mutation of valine at position 225 to isoleucine (V225I); the amino acid substitution of the M protein further includes mutation of glycine at position 21 to glutamate (G21E); and/or, the amino acid substitution of the M protein includes mutation of methionine at position 51 to arginine (M51R); and/or, mutation of methionine at position 51 to alanine (M51A); and/or, mutation of leucine at position 111 to alanine (L111A); and/or, mutation of valine at position 221 to phenylalanine (V221F); and/or, mutation of serine at position 226 to arginine (S226R).

In the present application, the M protein may include an amino acid mutation at position 21.

In the present application, the M protein may include amino acid mutations at positions 21 and 32.

In the present application, the M protein may include amino acid mutations at positions 21, 32 and 33.

In the present application, the M protein may include amino acid mutations at positions 21, 32, 33 and 49.

In the present application, the M protein may include amino acid mutations at positions 21, 32, 33, 49, and 54.

In the present application, the M protein may include amino acid mutations at positions 21, 32, 33, 49, 54, and 111.

In the present application, the M protein may include amino acid mutations at positions 21, 32, 33, 49, 54, 111, and 133.

In the present application, the M protein may include amino acid mutations at positions 21, 32, 33, 49, 54, 111, 133, and 225.

In the present application, the M protein may include amino acid mutations at positions 21, 32, 33, 49, 51, 54, 111, 133, and 225.

In the present application, the M protein may include amino acid mutations at positions 21, 32, 33, 49, 51, 54, 111, 133, 221, and 225.

In the present application, the M protein may include amino acid mutations at positions 21, 32, 33, 49, 51, 54, 111, 133, 221, 225, and 226.

In the present application, the M protein may include amino acid mutations at positions 32, 33, 49, 51, 54, 111, 133, 221, 225, and 226.

In the present application, the M protein may include amino acid mutations at positions 33, 49, 51, 54, 111, 133, 221, 225, and 226.

In the present application, the M protein may include amino acid mutations at positions 49, 51, 54, 111, 133, 221, 225, and 226.

In the present application, the M protein may include amino acid mutations at positions 51, 54, 111, 133, 221, 225, and 226.

In the present application, the M protein may include amino acid mutations at positions 54, 111, 133, 221, 225, and 226.

In the present application, the M protein may include amino acid mutations at positions 111, 133, 221, 225, and 226.

In the present application, the M protein may include amino acid mutations at positions 133, 221, 225 and 226.

In the present application, the M protein may include amino acid mutations at positions 221, 225 and 226.

In the present application, the M protein may include amino acid mutations at positions 225 and 226.

In the present application, the M protein may include an amino acid mutation at position 226.

In the present application, the M protein may include amino acid mutations at positions 32, 49, 54 and 225.

In the present application, the M protein may include amino acid mutations at positions 32, 49, 54, 225, and 226.

In the present application, the M protein may include amino acid mutations at positions 32, 49, 51, 54, 221, 225, and 226.

In the present application, the M protein may include amino acid mutations at positions 32, 33, 49, 51, 54, 221, 225, and 226.

In the present application, the M protein may include amino acid mutations at positions 32, 49, 51, 54, 133, 221, 225, and 226.

In the present application, the M protein may include amino acid mutations at positions 32, 33, 49, 51, 54, 133, 221, 225, and 226.

In the present application, the M protein may include amino acid mutations at positions 21, 32, 49, 51, 54, 111, 225, and 226.

In the present application, the M protein may further include amino acid mutations at other positions.

The oncolytic virus further includes a G protein, wherein the G protein includes amino acid substitution(s) at one or more of the following positions compared to an amino acid sequence set forth in SEQ ID NO 31: position 438, position 453, position 471, and position 487.

In the present application, the amino acid substitution of the G protein includes mutation of valine at position 53 to isoleucine (V53I); and/or mutation of alanine at position 141 to valine (A141V); and/or mutation of aspartic acid at position 172 to tyrosine (D172Y); and/or mutation of lysine at position 217 to glutamic acid (K217E); and/or mutation of aspartic acid at position 232 to glycine (D232G); and/or mutation of valine at position 331 to alanine (V331A); and/or, mutation of valine at position 371 to glutamic acid (V371E); and/or, mutation of glycine at position 436 to aspartic acid (G436D); and/or, mutation of threonine at position 438 to serine (T438S); and/or, mutation of phenylalanine at position 453 to leucine (F453L); and/or, mutation of threonine at position 471 to isoleucine (T471I); and/or, mutation of tyrosine at position 487 to histidine (Y487H). For example, the G protein includes an amino acid sequence as set forth in SEQ ID NO 43.

In the present application, the G protein may include an amino acid mutation at position 53.

In the present application, the G protein may include amino acid mutations at positions 53 and 141.

In the present application, the G protein may include amino acid mutations at positions 53, 141 and 172.

In the present application, the G protein may include amino acid mutations at positions 53, 141, 172, and 217.

In the present application, the G protein may include amino acid mutations at positions 53, 141, 172, 217, and 232.

In the present application, the G protein may include amino acid mutations at positions 53, 141, 172, 217, 232, and 331.

In the present application, the G protein may include amino acid mutations at positions 53, 141, 172, 217, 232, 331, and 371.

In the present application, the G protein may include amino acid mutations at positions 53, 141, 172, 217, 232, 331, 371, and 436.

In the present application, the G protein may include amino acid mutations at positions 53, 141, 172, 217, 232, 331, 371, 436, and 438.

In the present application, the G protein may include amino acid mutations at positions 53, 141, 172, 217, 232, 331, 371, 436, 438, and 453.

In the present application, the G protein may include amino acid mutations at positions 53, 141, 172, 217, 232, 331, 371, 436, 438, 453, and 471.

In the present application, the G protein may include amino acid mutations at positions 53, 141, 172, 217, 232, 331, 371, 436, 438, 453, 471, and 487.

In the present application, the G protein may include amino acid mutations at positions 141, 172, 217, 232, 331, 371, 436, 438, 453, 471, and 487.

In the present application, the G protein may include amino acid mutations at positions 172, 217, 232, 331, 371, 436, 438, 453, 471, and 487.

In the present application, the G protein may include amino acid mutations at positions 217, 232, 331, 371, 436, 438, 453, 471, and 487.

In the present application, the G protein may include amino acid mutations at positions 232, 331, 371, 436, 438, 453, 471, and 487.

In the present application, the G protein may include amino acid mutations at positions 331, 371, 436, 438, 453, 471, and 487.

In the present application, the G protein may include amino acid mutations at positions 371, 436, 438, 453, 471, and 487.

In the present application, the G protein may include amino acid mutations at positions 436, 438, 453, 471, and 487.

In the present application, the G protein may include amino acid mutations at positions 438, 453, 471, and 487.

In the present application, the G protein may include amino acid mutations at positions 453, 471 and 487.

In the present application, the G protein may include amino acid mutations at positions 471 and 487.

In the present application, the G protein may include an amino acid mutation at position 487.

In the present application, the G protein may further include amino acid mutations at other positions.

In some embodiments, the G protein at least includes one or more amino acid substitutions in a conserved region. For example, the conserved region may include amino acids of the G protein at positions 437-461. In some embodiments, the G protein at least includes one or more amino acid substitutions in a truncated region of a cytoplasmic domain. For example, the truncated region of the cytoplasmic domain may include amino acids of the G protein at positions 483-511.

In the present application, the G protein may at least include amino acid substitutions at positions 438, 453, 471 and 487.

The oncolytic virus further includes an N protein, wherein the N protein includes amino acid substitution(s) at one or more of the following positions compared to an amino acid sequence set forth in SEQ ID NO 55: position 14, position 155, and position 353.

In the present application, the amino acid substitution of the N protein includes mutation of isoleucine at position 14 to valine (I14V); and/or, mutation of arginine at position 155 to lysine (R155K); and/or, mutation of serine at position 353 to asparagine (S353N). For example, the N protein includes an amino acid sequence as set forth in SEQ ID NO 58.

In the present application, the N protein may include an amino acid mutation at position 14.

In the present application, the N protein may include amino acid mutations at positions 14 and 155.

In the present application, the N protein may include amino acid mutations at positions 14, 155 and 353.

In the present application, the N protein may include amino acid mutations at positions 155 and 353.

In the present application, the N protein may include an amino acid mutation at position 353.

In the present application, the N protein may further include amino acid substitutions at other positions.

The oncolytic virus further includes a P protein, wherein the P protein includes amino acid substitution(s) at one or more of the following positions compared to an amino acid sequence set forth in SEQ ID NO 61: position 50, position 76, position 99, position 126, position 140, position 151, position 168, position 170, position 189, and position 237.

In the present application, the amino acid substitution of the P protein includes mutation of arginine at position 50 to lysine (R50K); and/or mutation of valine at position 76 to alanine (V76A); and/or mutation of asparagine at position 99 to glutamic acid (D99E); and/or mutation of leucine at position 126 to serine (L126S); and/or mutation of leucine at position 140 to serine (L140S); and/or mutation of histidine at position 151 to tyrosine (H151Y); and/or mutation of isoleucine at position 168 to methionine (I168M); and/or mutation of lysine at position 170 to glutamic acid (K170E); and/or mutation of tyrosine at position 189 to serine (Y189S); and/or mutation of asparagine at position 237 to aspartic acid (N237D). For example, the P protein includes an amino acid sequence as set forth in SEQ ID NO 71.

In the present application, the P protein may include an amino acid mutation at position 50.

In the present application, the P protein may include amino acid mutations at positions 50 and 76.

In the present application, the P protein may include amino acid mutations at positions 50, 76 and 99.

In the present application, the P protein may include amino acid mutations at positions 50, 76, 99, and 126.

In the present application, the P protein may include amino acid mutations at positions 50, 76, 99, 126, and 140.

In the present application, the P protein may include amino acid mutations at positions 50, 76, 99, 126, 140, and 151.

In the present application, the P protein may include amino acid mutations at positions 50, 76, 99, 126, 140, 151, and 168.

In the present application, the P protein may include amino acid mutations at positions 50, 76, 99, 126, 140, 151, 168, and 170.

In the present application, the P protein may include amino acid mutations at positions 50, 76, 99, 126, 140, 151, 168, 170, and 189.

In the present application, the P protein may include amino acid mutations at positions 50, 76, 99, 126, 140, 151, 168, 170, 189, and 237.

In the present application, the P protein may include amino acid mutations at positions 76, 99, 126, 140, 151, 168, 170, 189, and 237.

In the present application, the P protein may include amino acid mutations at positions 99, 126, 140, 151, 168, 170, 189, and 237.

In the present application, the P protein may include amino acid mutations at positions 126, 140, 151, 168, 170, 189, and 237.

In the present application, the P protein may include amino acid mutations at positions 140, 151, 168, 170, 189, and 237.

In the present application, the P protein may include amino acid mutations at positions 151, 168, 170, 189, and 237.

In the present application, the P protein may include amino acid mutations at positions 168, 170, 189, and 237.

In the present application, the P protein may include amino acid mutations at positions 170, 189 and 237.

In the present application, the P protein may include amino acid mutations at positions 189 and 237.

In the present application, the P protein may include an amino acid mutation at position 237.

In the present application, the P protein may further include amino acid substitutions at other positions.

The oncolytic virus further includes an L protein, wherein the L protein includes amino acid substitution(s) at one or more of the following positions compared to an amino acid sequence set forth in SEQ ID NO 81: position 87 and position 487. For example, the L protein includes an amino acid sequence as set forth in SEQ ID NO 83.

In some specific embodiments, the amino acid substitution of the L protein includes mutation of serine at position 87 to proline (S87P); and/or mutation of isoleucine at position 487 to threonine (I487T).

In the present application, the L protein may include an amino acid mutation at position 87.

In the present application, the L protein may include amino acid mutations at positions 87 and 487.

In the present application, the L protein may include an amino acid mutation at position 487.

In the present application, the L protein may further include amino acid substitutions at other positions.

The oncolytic virus may further include a nucleic acid molecule and an foreign target protein. The nucleic acid molecule includes a nucleic acid sequence encoding the M protein with the amino acid substitution(s), and/or a nucleic acid sequence encoding the G protein with the amino acid substitution(s), and/or a nucleic acid sequence encoding the N protein with the amino acid substitution(s), and/or a nucleic acid sequence encoding the P protein with the amino acid substitution(s), and/or a nucleic acid sequence encoding the L protein with the amino acid substitution(s).

Further, the nucleic acid molecule includes a nucleic acid sequence encoding the foreign target protein. The nucleic acid sequence encoding the foreign target protein is positioned between the nucleic acid sequence encoding the M protein with the amino acid substitution(s), and/or the nucleic acid sequence encoding the G protein with the amino acid substitution(s), and/or the nucleic acid sequence encoding the N protein with the amino acid substitution(s), and/or the nucleic acid sequence encoding the P protein with the amino acid substitution(s), and/or the nucleic acid sequence encoding the L protein with the amino acid substitution(s).

In the present application, the oncolytic virus of the present application may be obtained by a virus packaging process and a virus rescue process. The specific process may include: infecting and inoculating a BSR-T7 cell with a poxvirus vTF7-3 expressing a T7 RNA polymerase, and performing lipofectamine transfection by using expression plasmids and backbone plasmids that clone VSVN, VSVP, and VSVL genes, respectively, to obtain target oncolytic viruses.

The present application further provides an oncolytic virus expression vector, a virus production cell, and a pharmaceutical composition.

The oncolytic virus expression vector may include a nucleic acid sequence encoding the M protein of the oncolytic virus and a nucleic acid sequence encoding the G protein of the oncolytic virus; the oncolytic virus expression vector may further include a nucleic acid sequence encoding the N protein of the oncolytic virus, a nucleic acid sequence encoding the P protein of the oncolytic virus, and a nucleic acid sequence encoding the L protein of the oncolytic virus.

The virus production cell is capable of producing the oncolytic virus described above; the virus production cell may includes BSR-T7 cell, Vero cell, 293 cell, MRC-5 cell, and WI38 cell.

The pharmaceutical composition includes the oncolytic virus described above, and optionally a pharmaceutically acceptable carrier.

In certain embodiments, the pharmaceutical composition may include a suitable formulation of one or more (pharmaceutically effective) adjuvants, stabilizers, excipients, diluents, solubilizers, surfactants, emulsifiers and/or preservatives. Acceptable components of the pharmaceutical composition are preferably nontoxic to recipients at the dosages and concentrations employed. The pharmaceutical composition of the present application includes, but is not limited to, liquid, frozen and lyophilized compositions.

In this application, the pharmaceutically acceptable carrier may include any and all solvents, dispersion media, coatings, isotonic agents and absorption delaying agents compatible with drug administration, which are typically safe and non-toxic.

The pharmaceutical composition includes the oncolytic virus described above and, optionally, other pharmaceutically acceptable drugs.

The described pharmaceutical composition can be used for combined treatment of a disease, including but not limited to the treatment of a tumor.

In certain embodiments, the pharmaceutical composition may be useful for administration including parenteral, subcutaneous, intracavitary, intravenous, intrathecal, and/or intranasal administration or may be directly injected into tissues. For example, the pharmaceutical composition may be administered to a patient or subject by infusion or injection. In certain embodiments, the administration of the pharmaceutical composition may be performed by various routes, such as an intravenous, intraperitoneal, subcutaneous, intramuscular, topical or intradermal route. In certain embodiments, the pharmaceutical composition may be administered uninterruptedly. The uninterrupted (or continuous) administration may be achieved by a small pump system worn by a patient to measure a therapeutic agent flowing into the patient's body, as described in WO2015/036583.

In addition, the present application further provides a method for preparing the described oncolytic virus, and the method may include a method for preparing an oncolytic virus expression vector, a virus production cell and/or a pharmaceutical composition. Any method suitable for producing an oncolytic virus may be used to produce the oncolytic virus of the present application. For example, the oncolytic virus of the present application may be obtained by transfecting a cell with a poxvirus expressing a T7 RNA polymerase, performing transfection with plasmids expressing the N protein, L protein and P protein of an oncolytic virus and backbone plasmids and performing a virus rescue process.

The present application further provides a use of the described oncolytic virus, the described oncolytic virus expression vector, the described virus production cell and/or the described pharmaceutical composition in the preparation of a drug for prevention and/or treatment of a disease and/or disorder.

The oncolytic viruses of the present application are obtained by performing site-directed mutagenesis on the amino acids on the M protein, G protein, N protein, P protein and L protein of an oncolytic virus, respectively, thereby further improving the infection ability of the oncolytic virus to the abnormally proliferative (tumor) LLC cell, 4T1 cell, MC38 cell and Hela cell. Moreover, the oncolytic viruses prepared have poor infection ability to normal cells and the normal MEF cell, indicating that the oncolytic viruses prepared in the present application can be well used for infecting tumors, cancers and other cells without damaging normal cells, and have broad application prospects.

The oncolytic viruses of the present application are obtained by performing site-directed mutagenesis on the amino acids on the M protein, G protein, N protein, P protein and L protein of an oncolytic virus, respectively, thereby further improving the infection ability of the oncolytic virus to the abnormally proliferative (tumor) LLC cell, 4T1 cell, MC38 cell and Hela cell. and further improving the in vitro cytotoxicity of the oncolytic virus against the LLC cell, the 4T1 cell, the MC38 cell and the Hela cell. Moreover, the oncolytic viruses prepared have almost no effect on the normal MEF cell, indicating that the oncolytic viruses prepared herein can be well used for damaging and killing abnormal cells such as tumor and cancer cells, without causing damage to normal cells.

The oncolytic viruses of the present application are not easily cleared from the abnormally proliferative (tumor) LLC cell, 4T1 cell, MC38 cell and Hela cell. Relatively speaking, wild-type oncolytic viruses are more easily cleared from the LLC cell, the MC38 cell and the Hela cell. The oncolytic viruses of the present application are obtained by performing site-directed mutagenesis on the amino acids on the M protein, G protein, N protein, P protein, and L protein of an oncolytic virus, respectively, so that the oncolytic viruses are more difficultly cleared from the LLC cell, the 4T1 cell, the MC38 cell, and the Hela cell, thereby further ensuring that the oncolytic viruses can better exert their infection ability and cytotoxicity to the LLC cell, the 4T1 cell, the MC38 cell, and the Hela cell. Moreover, the oncolytic viruses of the present application are more easily cleared from the normal MEF cell and the safety of the normal MEF cell is further ensured, thereby improving the safety of the oncolytic viruses.

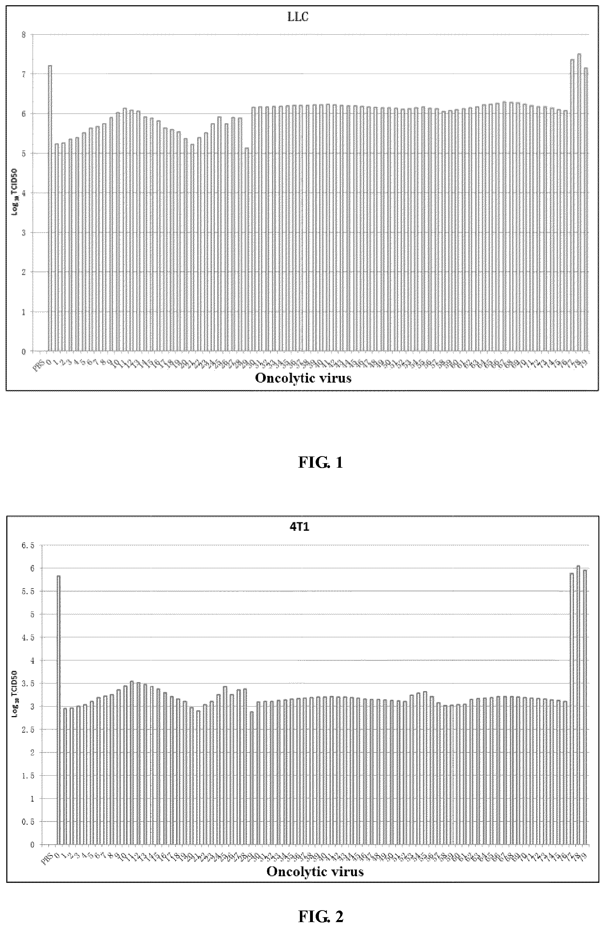

The present application is further described in detail in conjunction with Preparation Examples 1-80, Examples 1-3, and FIGS. 1-15.

### PREPARATION EXAMPLES

### Preparation Examples 1-29

Preparation Examples 1-29 respectively provide an oncolytic virus, and they mainly differ in the positions of amino acids having site-directed mutagenesis on the M protein of a wild-type oncolytic virus. A construction method for the oncolytic virus corresponding to each preparation example is as follows:

### (1) Construction of plasmids

Using a pRV-core plasmid (BioVector NTCC Plasmid Vector Strain Cell Gene Collection Center) as a template, mutation sites shown in Table 1 were introduced by a PCR technology. The pRV-core plasmid was subjected to PCR with primers carrying the mutation sites; then, the PCR product was subjected to 1% agarose gel electrophoresis; and then a gel recovery kit was used to cut and recover the gel, thereby obtaining a plasmid with different mutation sites on the M protein and obtaining a constructed plasmid pRV-core Mut.

**Table 1 Mutations on the M protein of the oncolytic viruses in Preparation Examples 1-29**

| Preparation Example | Mutation position and amino acid after mutation | Number of mutation positions | Amino acid sequence |
|---|---|---|---|
| 1 | G21E | 1 | SEQ ID NO 2 |
| 2 | G21E, N32S | 2 | SEQ ID NO 3 |
| 3 | G21E, N32S, M33A | 3 | SEQ ID NO 4 |
| 4 | G21E, N32S, M33A, N49D | 4 | SEQ ID NO 5 |
| 5 | G21E, N32S, M33A, N49D, H54Y | 5 | SEQ ID NO 6 |
| 6 | G21E, N32S, M33A, N49D, H54Y, L111A | 6 | SEQ ID NO 7 |
| 7 | G21E, N32S, M33A, N49D, H54Y, L111A, A133T | 7 | SEQ ID NO 8 |
| 8 | G21E, N32S, M33A, N49D, H54Y, L111A, A133T, V225I | 8 | SEQ ID NO 9 |
| 9 | G21E, N32S, M33A, N49D, M51R, H54Y, L111A, A133T, V225I | 9 | SEQ ID NO 10 |
| 10 | G21E, N32S, M33A, N49D, M51R, H54Y, L111A, A133T, V221F, V225I | 10 | SEQ ID NO 11 |
| 11 | G21E, N32S, M33A, N49D, M51R, H54Y, L111A, A133T, V221F, V225I, S226R | 11 | SEQ ID NO 12 |
| 12 | N32S, M33A, N49D, M51R, H54Y, L111A, A133T, V221F, V225I, S226R | 10 | SEQ ID NO 13 |
| 13 | M33A, N49D, M51R, H54Y, L111A, A133T, V221F, V225I, S226R | 9 | SEQ ID NO 14 |
| 14 | N49D, M51R, H54Y, L111A, A133T, V221F, V225I, S226R | 8 | SEQ ID NO 15 |
| 15 | M51R, H54Y, L111A, A133T, V221F, V225I, S226R | 7 | SEQ ID NO 16 |
| 16 | H54Y, L111A, A133T, V221F, V225I, S226R | 6 | SEQ ID NO 17 |
| 17 | L111A, A133T, V221F, V225I, S226R | 5 | SEQ ID NO 18 |
| 18 | A133T, V221F, V225I, S226R | 4 | SEQ ID NO 19 |
| 19 | V221F, V225I, S226R | 3 | SEQ ID NO 20 |
| 20 | V225I, S226R | 2 | SEQ ID NO 21 |
| 21 | S226R | 1 | SEQ ID NO 22 |
| 22 | N32S, N49D, H54Y, V225I | 4 | SEQ ID NO 23 |
| 23 | N32S, N49D, H54Y, V225I, S226G | 5 | SEQ ID NO 24 |
| 24 | N32S, N49D, M51R, H54Y, V221F, V225I, S226R | 7 | SEQ ID NO 25 |
| 25 | N32S, M33A, N49D, M51R, H54Y, V221F, V225I, S226R | 8 | SEQ ID NO 26 |
| 26 | N32S, N49D, M51R, H54Y, A133T, V221F, V225I, S226R | 8 | SEQ ID NO 27 |
| 27 | N32S, M33A, N49D, M51R, H54Y, A133T, V221F, V225I, S226R | 9 | SEQ ID NO 28 |
| 28 | G21E, N32S, N49D, M51A, H54Y, L111A, V225I, S226R | 8 | SEQ ID NO 29 |
| 29 | M51R, V221F, S226R | 3 | SEQ ID NO 30 |

### (2) Virus rescue

The constructed plasmid pRV-core Mut was transfected into a BSR-T7 cell (purchased from ATCC, American Type Culture Collection) using a calcium phosphate transfection kit (Thermo Fisher Scientific) by a cell transfection technology.

Four plasmids, i.e., the pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rod-shaped virus, the pN was a plasmid carrying the nucleoprotein genes of a rod-shaped virus, and the pL was a plasmid carrying the polymerase protein genes of a rod-shaped virus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

Plasmid transfection was performed according to the method described in the instruction manual of Lipofectamine LTX. Six hours later, the BSR-T7 cell was washed twice with PBS and further inoculated in DMEM (Thermo Fisher Scientific) containing 10% fetal bovine serum and incubated for three days.

The cell supernatant of the BSR-T7 cell culture solution was transferred to a Vero cell (Thermo Fisher Scientific), and the Vero cell was incubated at 37 °C for three days. The cells were observed for green fluorescence under a fluorescence microscope to determine the result of virus rescue. The rescued mutant rod-shaped virus library was further passaged through the Vero cell, and monoclonal virus strains were selected in an established plaque screening system.

(3) Gene sequencing of the M protein The viral genomic RNA was extracted using a Trizol kit, and reverse transcription was performed using random primers. The reverse transcribed cDNA was subjected to PCR using primers designed for the gene sequence of the M protein.

the primer sequences were:
□ PF: ATGAGTTCCTTAAAGAA;
□ PR: TCATTTGAAGTGG

The product was recovered after 1% agarose gel electrophoresis and sent to a sequencing company for sequencing. The sequencing results are shown in Table 1.

### Preparation Examples 30-52

Preparation Examples 30-52 respectively provide an oncolytic virus, and they mainly differ in the positions of amino acids having site-directed mutagenesis on the M protein and G protein of a wild-type oncolytic virus. The mutation sites of the M protein are the same as the corresponding mutation sites in Preparation Example 24, and the mutation sites of the G protein are shown in Table 2.

The construction methods for the oncolytic viruses in these preparation examples are the same as the construction method in Preparation Example 11, except that:
the mutation sites shown in Table 2 were introduced, by a PCR technology, into the plasmids constructed in step (1); and
gene sequencing of the G protein was performed in step (3), where the viral genomic RNA was extracted using a Trizol kit, and reverse transcription was performed using random primers; the reverse transcribed cDNA was subjected to PCR using primers designed for the gene sequence of the G protein;
the primer sequences were:
   □ PF: ATGAAGTGCCTTTTGTACTTAG;
   □ PR: TTACTTTCCAAGTCGGTTCATCT.

The product was recovered after 1% agarose gel electrophoresis and sent to a sequencing company for sequencing. The sequencing results are shown in Table 2.

**Table 2 Mutations on the G protein of the oncolytic viruses in Preparation Examples 30-52**

| Preparation Example | Mutation position and amino acid after mutation | Number of mutation positions | Amino acid sequence |
|---|---|---|---|
| 30 | V53I | 1 | SEQ ID NO 32 |
| 31 | V53I, A141V | 2 | SEQ ID NO 33 |
| 32 | V53I, A141V, D172Y | 3 | SEQ ID NO 34 |
| 33 | V53I, A141V, D172Y, K217E | 4 | SEQ ID NO 35 |
| 34 | V53I, A141V, D172Y, K217E, D232G | 5 | SEQ ID NO 36 |
| 35 | V53I, A141V, D172Y, K217E, D232G , V331A | 6 | SEQ ID NO 37 |
| 36 | V53I, A141V, D172Y, K217E, D232G , V331A, V371E | 7 | SEQ ID NO 38 |
| 37 | V53I, A141V, D172Y, K217E, D232G , V331A, V371E, G436D | 8 | SEQ ID NO 39 |
| 38 | V53I, A141V, D172Y, K217E, D232G , V331A, V371E, G436D, T438S | 9 | SEQ ID NO 40 |
| 39 | V53I, A141V, D172Y, K217E, D232G , V331A, V371E, G436D, T438S, F453L | 10 | SEQ ID NO 41 |
| 40 | V53I, A141V, D172Y, K217E, D232G , V331A, V371E, G436D, T438S, F453L, T471I | 11 | SEQ ID NO 42 |
| 41 | V53I, A141V, D172Y, K217E, D232G , V331A, V371E, G436D, T438S, F453L, T471I, Y487H | 12 | SEQ ID NO 43 |
| 42 | A141V, D172Y, K217E, D232G, V331A, V371E, G436D, T438S, F453L , T471I, Y487H | 11 | SEQ ID NO 44 |
| 43 | D172Y, K217E, D232G, V331A, V371E , G436D, T438S, F453L, T471I, Y487H | 10 | SEQ ID NO 45 |
| 44 | K217E, D232G, V331A, V371E, G436D , T438S, F453L, T471I, Y487H | 9 | SEQ ID NO 46 |
| 45 | D232G, V331A, V371E, G436D, T438S , F453L, T471I, Y487H | 8 | SEQ ID NO 47 |
| 46 | V331A, V371E, G436D, T438S, F453L , T471I, Y487H | 7 | SEQ ID NO 48 |
| 47 | V371E, G436D, T438S, F453L, T471I , Y487H | 6 | SEQ ID NO 49 |
| 48 | G436D, T438S, F453L, T471I, Y487H | 5 | SEQ ID NO 50 |
| 49 | T438S, F453L, T471I, Y487H | 4 | SEQ ID NO 51 |
| 50 | F453L, T471I, Y487H | 3 | SEQ ID NO 52 |
| 51 | T471I, Y487H | 2 | SEQ ID NO 53 |
| 52 | Y487H | 1 | SEQ ID NO 54 |

### Preparation Examples 53-57

Preparation Examples 53-57 respectively provide an oncolytic virus, and they mainly differ in the positions of amino acids having site-directed mutagenesis on the M protein, G protein and N protein of a wild-type oncolytic virus. The mutation sites of the M protein and the G protein are the same as the corresponding mutation sites in Preparation Example 41, and the mutation sites of the N protein are shown in Table 3.

The construction methods for the oncolytic viruses in these preparation examples are the same as the construction method in Preparation Example 41, except that:
the mutation sites shown in Table 3 were introduced, by a PCR technology, into the plasmids constructed in step (1); and
gene sequencing of the N protein was performed in step (3), where the viral genomic RNA was extracted using a Trizol kit, and reverse transcription was performed using random primers; the reverse transcribed cDNA was subjected to PCR using primers designed for the gene sequence of the N protein;
the primer sequences were:
   □ PF: ATGTCTGTTACAGTCAAGAG;
   □ PR: TCATTTGTCAAATTCTGACTT.

The product was recovered after 1% agarose gel electrophoresis and sent to a sequencing company for sequencing. The sequencing results are shown in Table 3.

**Table 3 Mutations on the N protein of the oncolytic viruses in Preparation Examples 53-57**

| Preparation Example | Mutation position and amino acid after mutation | Number of mutation positions | Amino acid sequence |
|---|---|---|---|
| 53 | I14V | 1 | SEQ ID NO 56 |
| 54 | I14V, R155K | 2 | SEQ ID NO 57 |
| 55 | I14V, R155K, S353N | 3 | SEQ ID NO 58 |
| 56 | R155K, S353N | 2 | SEQ ID NO 59 |
| 57 | S353N | 1 | SEQ ID NO 60 |

### Preparation Examples 58-76

Preparation Examples 58-76 respectively provide an oncolytic virus, and they mainly differ in the positions of amino acids having site-directed mutagenesis on the M protein, G protein, N protein, and P protein of a wild-type oncolytic virus. The mutation sites of the M protein, the G protein and the N protein are the same as the corresponding mutation sites in Preparation Example 55, and the mutation sites of the P protein are shown in Table 4.

The construction methods for the oncolytic viruses in these preparation examples are the same as the construction method in Preparation Example 55, except that:
the mutation sites shown in Table 4 were introduced, by a PCR technology, into the plasmids constructed in step (1); and
gene sequencing of the P protein was performed in step (3), where the viral genomic RNA was extracted using a Trizol kit, and reverse transcription was performed using random primers; the reverse transcribed cDNA was subjected to PCR using primers designed for the gene sequence of the P protein;
the primer sequences were:
   □ PF: ATGGATAATCTCACAAAAGTTCG;
   □ PR: CTACAGAGAATATTTGACTCTCG

The product was recovered after 1% agarose gel electrophoresis and sent to a sequencing company for sequencing. The sequencing results are shown in Table 4.

**Table 4 Mutations on the P protein of the oncolytic viruses in Preparation Examples 58-76**

| Preparation Example | Mutation position and amino acid after mutation | Number of mutation positions | Amino acid sequence |
|---|---|---|---|
| 58 | R50K | 1 | SEQ ID NO 62 |
| 59 | R50K, V76A | 2 | SEQ ID NO 63 |
| 60 | R50K, V76A, D99E | 3 | SEQ ID NO 64 |
| 61 | R50K, V76A, D99E, L126S | 4 | SEQ ID NO 65 |
| 62 | R50K, V76A, D99E, L126S, L140S | 5 | SEQ ID NO 66 |
| 63 | R50K, V76A, D99E, L126S, L140S, H151Y | 6 | SEQ ID NO 67 |
| 64 | R50K, V76A, D99E, L126S, L140S, H151Y, I168M | 7 | SEQ ID NO 68 |
| 65 | R50K, V76A, D99E, L126S, L140S, H151Y, I168M, K170E | 8 | SEQ ID NO 69 |
| 66 | R50K, V76A, D99E, L126S, L140S, H151Y, I168M, K170E, Y189S | 9 | SEQ ID NO 70 |
| 67 | R50K, V76A, D99E, L126S, L140S, H151Y, I168M, K170E, Y189S, N237D | 10 | SEQ ID NO 71 |
| 68 | V76A, D99E, L126S, L140S, H151Y, I168M, K170E, Y189S, N237D | 9 | SEQ ID NO 72 |
| 69 | D99E, L126S, L140S, H151Y, I168M, K170E, Y189S, N237D | 8 | SEQ ID NO 73 |
| 70 | L126S, L140S, H151Y, I168M, K170E , Y189S, N237D | 7 | SEQ ID NO 74 |
| 71 | L140S, H151Y, I168M, K170E, Y189S , N237D | 6 | SEQ ID NO 75 |
| 72 | H151Y, I168M, K170E, Y189S, N237D | 5 | SEQ ID NO 76 |
| 73 | I168M, K170E, Y189S, N237D | 4 | SEQ ID NO 77 |
| 74 | K170E, Y189S, N237D | 3 | SEQ ID NO 78 |
| 75 | Y189S, N237D | 2 | SEQ ID NO 79 |
| 76 | N237D | 1 | SEQ ID NO 80 |

### Preparation Examples 77-79

Preparation Examples 77-79 respectively provide an oncolytic virus, and they mainly differ in the positions of amino acids having site-directed mutagenesis on the M protein, G protein, N protein, P protein, and L protein of a wild-type oncolytic virus. The mutation sites of the M protein, the G protein, the N protein and the P protein are the same as the corresponding mutation sites in Preparation Example 67, and the mutation sites of the L protein are shown in Table 5.

The construction methods for the oncolytic viruses in these preparation examples are the same as the construction method in Preparation Example 67, except that:
the mutation sites shown in Table 5 were introduced, by a PCR technology, into the plasmids constructed in step (1); and
gene sequencing of the L protein was performed in step (3), where the viral genomic RNA was extracted using a Trizol kit, and reverse transcription was performed using random primers; the reverse transcribed cDNA was subjected to PCR using primers designed for the gene sequence of the L protein;
the primer sequences were:
   □ PF: ATGGAAGTCCACGATTTTGAGA;
   □ PR: TTAATCTCTCCAAGAGTTTTCCT.

The product was recovered after 1% agarose gel electrophoresis and sent to a sequencing company for sequencing. The sequencing results are shown in Table 5.

**Table 5 Mutations on the L protein of the oncolytic viruses in Preparation Examples 77-79**

| Preparation Example | Mutation position and amino acid after mutation | Number of mutation positions | Amino acid sequence |
|---|---|---|---|
| 77 | S87P | 1 | SEQ ID NO 82 |
| 78 | S87P, I487T | 2 | SEQ ID NO 83 |
| 79 | I487T | 1 | SEQ ID NO 84 |

### Preparation Example 80

This preparation example provides a packaging process of the oncolytic virus prepared in any one of the preparation examples 1-79 described above, specifically including the following steps:

1) Infection and inoculation of a BSR-T7 cell (purchased from ATCC) with a poxvirus vTF7-3 (BioVector NTCC Plasmid Vector Strain Cell Gene Collection Center) expressing T7 RNA polymerase.

Specifically, the BSR-T7 cell was plated on a 6-well plate, with 3×10⁵ cells/well; 14-16 hours after plating, the poxvirus vTF7-3 expressing the T7 RNA polymerase was added to infect the BSR-T7 cell; 6 hours after infection, the BSR-T7 cell was rinsed once with a DPBS buffer for transfection later.

### 2) Transfection process

Specifically, four plasmids, i.e., pRV-core Mut, pP, pN, and pL, were mixed at a ratio of 10:5:4:1, and the total amount of the plasmids was 5 µg; the plasmid mixture was then diluted with 200 µl of an Opti-MEM (Thermo Fisher Scientific), and 7.5 µl of a transfection reagent Plus Reagent (Life Technologies) was added to obtain a transfection plasmid premix, where the pP was a plasmid carrying the phosphoprotein genes of a rod-shaped virus, the pN was a plasmid carrying the nucleoprotein genes of a rod-shaped virus, and the pL was a plasmid carrying the polymerase protein genes of a rod-shaped virus; parent vectors corresponding to the three plasmids pN, pP, and pL were all pCAGGS (purchased from ATCC).

10 µl of Lipofectamine LTX (Thermo Fisher Scientific) was diluted with 200 µl of the Opti-MEM to obtain an LTX mixed solution.

200 µl of the LTX mixture was mixed with 200 µl of the transfection plasmid premix and the cells were incubated at room temperature for 15 min to obtain an LTX-DNA mixed solution;

The DPBS buffer in the 6-well plate in step 1) was replaced with the Opti-MEM, the LTX-DNA mixed solution was dropwise added to the 6-well plate in which the BSR-T7 cell was incubated, and the 6-well plate was then shaken gently to evenly distribute the LTX-DNA mixed solution in the 6-well plate; 6-8 hours after transfection, the transfection reagent was removed and 3 ml of fresh complete medium (Thermo Fisher Scientific) was then added; 72 hours later, the cell supernatant of the BSR-T7 cell was harvested and filtered with a 0.22 µm filter to obtain the oncolytic viruses respectively corresponding to Preparation Examples 1-79.

### EXAMPLES

### Example 1

In this example, the oncolytic viruses prepared in Preparation Examples 1-79 and the wild-type oncolytic virus were tested for their infection ability to different cells.

The test method used was a TCID50 determination method, that is, 200 pfu of each of the oncolytic viruses prepared in Preparation Examples 1-79 and 200 pfu of the wild-type oncolytic virus were added to the culture solutions of different cells, and the 50% tissue culture infective dose (TCID50) of each oncolytic virus was then determined.

The cells tested included: LLC cell (a mouse lung cancer cell line), 4T1 cell (a mouse breast cancer cell line), MC38 cell (a mouse colon cancer cell line), Hela cell (a human cervical cancer cell line), and MEF cell (a human fibroblast cell line).

Test method:
(1) 3 mL of Vero (LLC/4T1/MC38/Hela/MEF) cell suspension was added to a 6-well plate, with 4×10⁵ cells/well, a total of 6 wells, including 2 wells for the MEF cell; the 6-well culture plate was then placed in an environment of 37 °C and 5% CO₂ for cell culture for 16 hours.
(2) 200 pfu of each of the oncolytic viruses prepared in the preparation examples was added to each well of the 6-well culture plate; 24 hours later, 100 µl of the supernatant of the MEF cell and 100 µl of the supernatant of each Vero cell were harvested; the harvested supernatants were added to the wells of a 96-well plate, with a density of each type of cell being 1×10⁴ cells /ml; and the 96-well plate was then placed in an environment of 37 °C and 5% CO₂ for cell culture for 16 hours.
(3) Each of the supernatants harvested in step (2) was diluted consecutive 10 folds in 1.5 ml EP tubes, from 10⁻¹ to 10⁻¹¹, with a total of 11 titers; the diluted supernatants were inoculated into 96-well culture plates, a row of 8 wells for each titer, 100 µl/well.
(4) 48 hours later, the cells in each well were observed for fluorescence, and the wells in which the cells were observed fluorescent were recorded as infected. TCID50 was calculated according to a Karber method.

The test results are shown in FIGS. 1-5. As shown in the figures, the number 0 on the horizontal axis represents the wild-type oncolytic virus; the numbers 1-79 on the horizontal axis represent the oncolytic viruses prepared in Preparation Examples 1-79, respectively; Log₁₀ TCID50 on the vertical axis represents the values of TCID50 calculated by the Karber method. The larger the value of Log₁₀ TCID50, the better the infection ability of an oncolytic virus to the cell; the smaller the value of Log₁₀ TCID50, the worse the infection ability of an oncolytic virus to the cell.

FIG. 1 shows test results of the oncolytic viruses of the present application and the wild-type oncolytic virus in infection ability to the LLC cell.

FIG. 2 shows test results of the oncolytic viruses of the present application and the wild-type oncolytic virus in infection ability to the 4T1 cell.

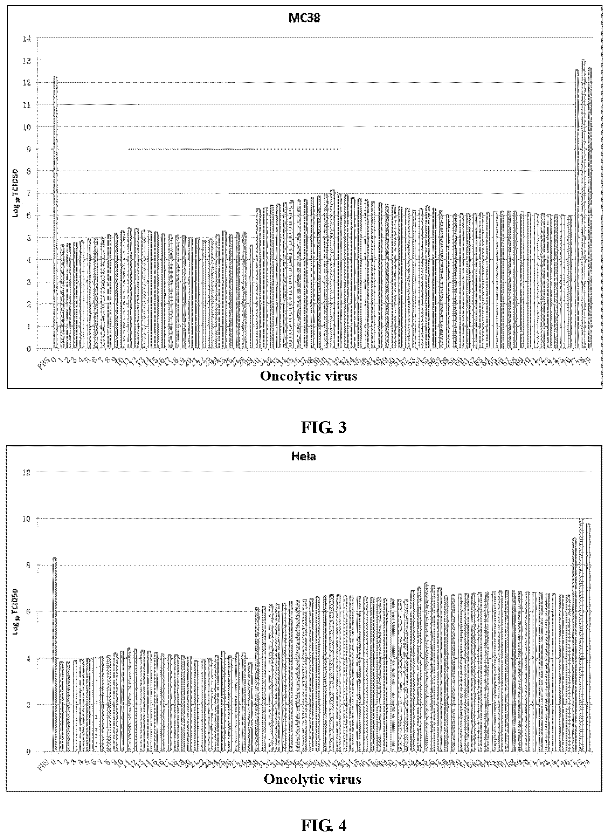

FIG. 3 shows test results of the oncolytic viruses of the present application and the wild-type oncolytic virus in infection ability to the MC38 cell.

FIG. 4 shows test results of the oncolytic viruses of the present application and the wild-type oncolytic virus in infection ability to the Hela cell.

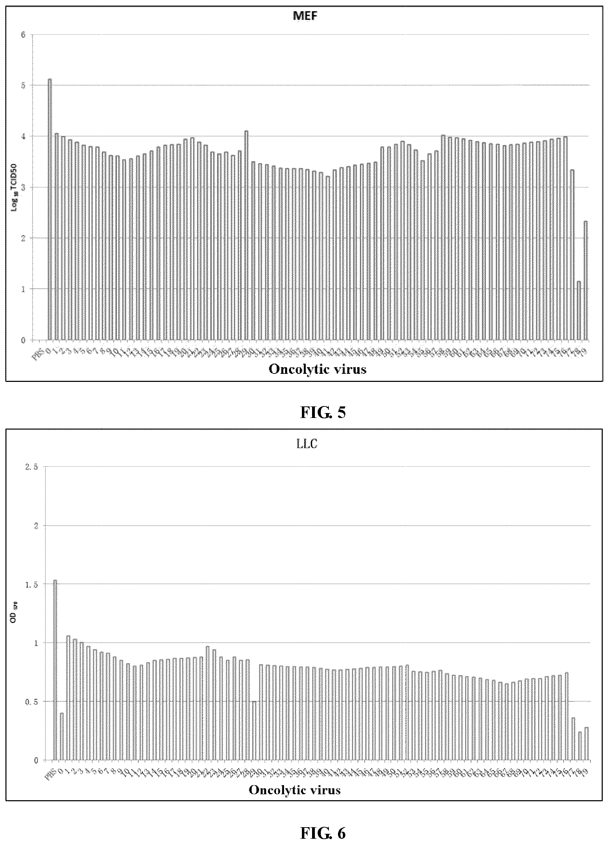

FIG. 5 shows test results of the oncolytic viruses of the present application and the wild-type oncolytic virus in infection ability to the MEF cell.

It can be seen from the above figures that the oncolytic viruses prepared in Preparation Examples 1-79 of the present application have good infection ability to the LLC cell, the 4T1 cell, the MC38 cell and the Hela cell, and especially the oncolytic viruses provided in Preparation Examples 77-79 have the same infection ability to the LLC cell, the 4T1 cell, the MC38 cell and the Hela cell as the wild-type oncolytic virus. Moreover, the oncolytic viruses prepared, especially the oncolytic viruses provided in Preparation Examples 77-79, all have poor infection ability to the MEF cell, indicating that the oncolytic viruses prepared in the present application can be well used for infecting tumors, cancers and other cells without damaging normal cells, and have broad application prospects.

### Example 2

In this example, the oncolytic viruses prepared in Preparation Examples 1-79 and the wild-type oncolytic virus were tested for their in vitro cytotoxicity against different cells.

The test method used was an MTT method, that is, 200 pfu of each of the oncolytic viruses prepared in Preparation Examples 1-79 and 200 pfu of the wild-type oncolytic virus were added to the culture solutions of different cells, and 24 hours later, cell viability was tested by using the MTT method.

The cells tested included: LLC cell, 4T1 cell, MC38 cell, Hela cell, and MEF cell.

Test method:
(1) 100 mL of Vero (LLC/4T1/MC38/Hela/MEF) cell suspension was added to each well of a 96-well plate, with 1×10⁴ cells/well; the 96-well culture plate was then placed in an environment of 37 °C and 5% CO₂ for cell culture for 16 hours.
(2) Each of the oncolytic virus prepared in the preparation examples was diluted to an MOI (multiplicity of infection) of 0.001, 0.01, 0.1, and 1.0, and the diluted oncolytic viruses were inoculated into the 96-well plate from step (1), 4 wells for each dilution gradient, 100 µl/well; and the 96-well plate was then placed in an environment of 37 °C and 5% CO₂ for cell culture for 40 hours.
(3) Cell supernatants were removed from the 96-well culture plate from step (2), fresh culture medium and MTT solution were then added to the 96-well plate with 20 µL/well; and the 96-well plate was then placed in an environment of 37 °C and 5% CO₂ for cell culture for 4 hours.
(4) The 96-well plate was centrifuged at room temperature, 2500 rpm/min for 5 min and the supernatants were gently removed with a 1 mL disposable sterile syringe; then, DMSO was added to the wells of the 96-well plate, 100 µl/well; and the 96-well plate was then placed in an environment of 37 °C for 10 min. The 96-well plate was shaken for 2 min on a multifunctional microplate reader, and the OD value of each well on the 96-well plate was measured at a wavelength of 570 nm or 490 nm.

The test results are shown in FIGS. 6-10. As shown in the figures, the number 0 on the horizontal axis represents the wild-type oncolytic virus; the numbers 1-79 on the horizontal axis represent the oncolytic viruses prepared in Preparation Examples 1-79, respectively; OD₅₇₀ on the vertical axis represents the OD values of the cells. The larger the value of OD₅₇₀, the worse the cytotoxicity of an oncolytic virus against the cell; the smaller the value of OD₅₇₀, the better the cytotoxicity of an oncolytic virus against the cell.

FIG. 6 shows test results of the oncolytic viruses of the present application and the wild-type oncolytic virus in in vitro cytotoxicity against the LLC cell.

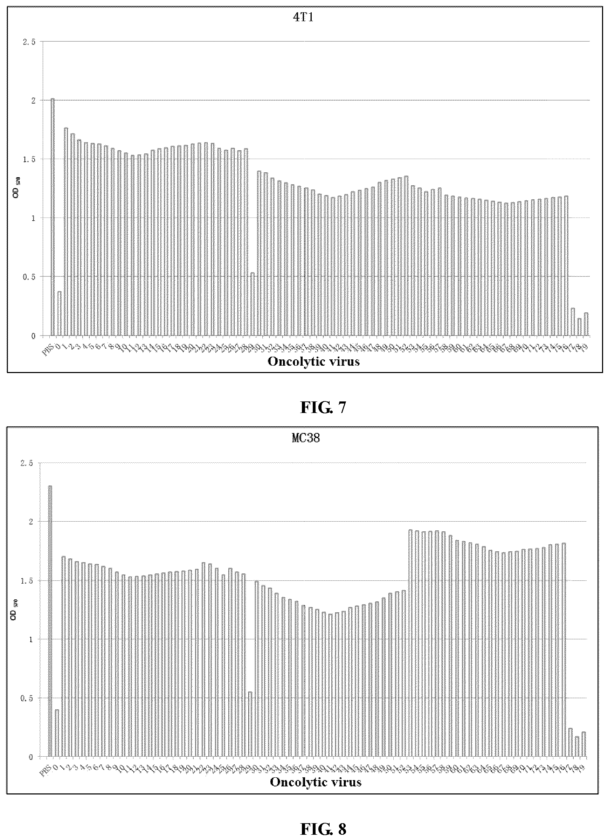

FIG. 7 shows test results of the oncolytic viruses of the present application and the wild-type oncolytic virus in in vitro cytotoxicity against the 4T1 cell.

FIG. 8 shows test results of the oncolytic viruses of the present application and the wild-type oncolytic virus in in vitro cytotoxicity against the MC38 cell.

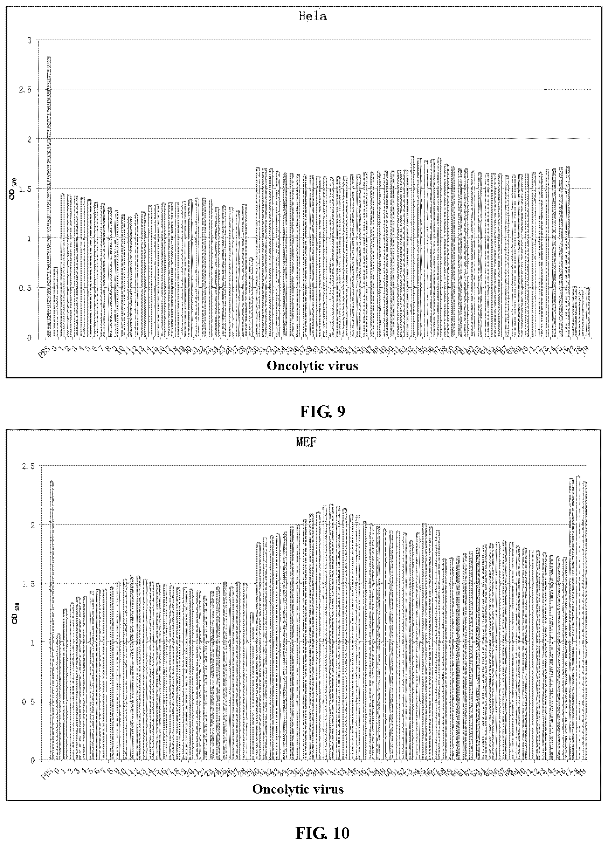

FIG. 9 shows test results of the oncolytic viruses of the present application and the wild-type oncolytic virus in in vitro cytotoxicity against the Hela cell.

FIG. 10 shows test results of the oncolytic viruses of the present application and the wild-type oncolytic virus in in vitro cytotoxicity against the MEF cell.

It can be seen from the above figures that the oncolytic viruses prepared in Preparation Examples 1-79 of the present application have good in vitro cytotoxicity against the LLC cell, the 4T1 cell, the MC38 cell and the Hela cell, and especially the oncolytic viruses provided in Preparation Examples 77-79 have better in vitro cytotoxicity against the LLC cell, the 4T1 cell, the MC38 cell and the Hela cell than the wild-type oncolytic virus. Moreover, the oncolytic viruses prepared have almost no cytotoxicity against the MEF cell, indicating that the oncolytic viruses of the present application can be well used for damaging and killing abnormal cells such as tumor and cancer cells, without causing damage to normal cells.

Although the wild-type oncolytic virus has good in vitro cytotoxicity against the LLC cell, the MC38 cell, and the 4T1 cell, while damaging and killing the above cells, the wild-type oncolytic virus will also damage and kill the MEF cell to a large extent at the same time, thereby limiting the clinical application of the wild-type oncolytic virus. Therefore, the modification of the wild-type oncolytic virus in the present application ensures the safety of oncolytic viruses to normal cells and the cytotoxicity of oncolytic viruses against tumor and cancer cells, thereby achieving broad clinical application prospects.

### Example 3

In this example, the expression of IFN-β induced by the oncolytic viruses prepared in Preparation Examples 1-79 and the wild-type oncolytic virus in different cells was tested.

The test indicator was the expression of the gene IFN-β in different cells. The gene IFN-β is a soluble glycoprotein gene produced by cells and having a wide range of antiviral, anti-tumor and immunomodulatory effects. The expression of the gene IFN-β can be used to determine the ability of cells to clear an oncolytic virus: when the expression of the gene IFN-β is high, it indicates that the oncolytic virus can be easily cleared from cells; when the expression of the gene IFN-β is low, it indicates that the oncolytic virus can be difficultly cleared from cells.

The cells tested included: LLC cell, 4T1 cell, MC38 cell, Hela cell, and MEF cell.

Test method:
(1) 100 mL of Vero (LLC/4T1/MC38/Hela/MEF) cell suspension was added to each well of a 96-well plate, with 1×10⁴ cells/well; the 96-well culture plate was then placed in an environment of 37 °C and 5% CO₂ for cell culture for 16 hours.
(2) Each of the oncolytic virus prepared in the preparation examples was diluted to an MOI (multiplicity of infection) of 0.001, 0.01, 0.1, and 1.0, and the diluted oncolytic viruses were inoculated into the 96-well plate from step (1), 4 wells for each dilution gradient, 100 µl/well; and the 96-well plate was then placed in an environment of 37 °C and 5% CO₂ for cell culture for 40 hours.
(3) Each group of cells obtained by culture in step (2) was broken, and total RNA was extracted from each cell using TRIzol (Invitrogen), reverse transcribed into cDNA by using a reverse transcription kit (PrimeScript RT Reagent Kit with DNA Eraser (Takara)), and stained with a dye (LightCycler 480SYBR Green I Master (Roche)), and the Ct value of each gene was detected on a LightCycler 480 quantitative PCR instrument. The relative expression level of the target gene IFN-β was calculated using a ΔΔCt method.

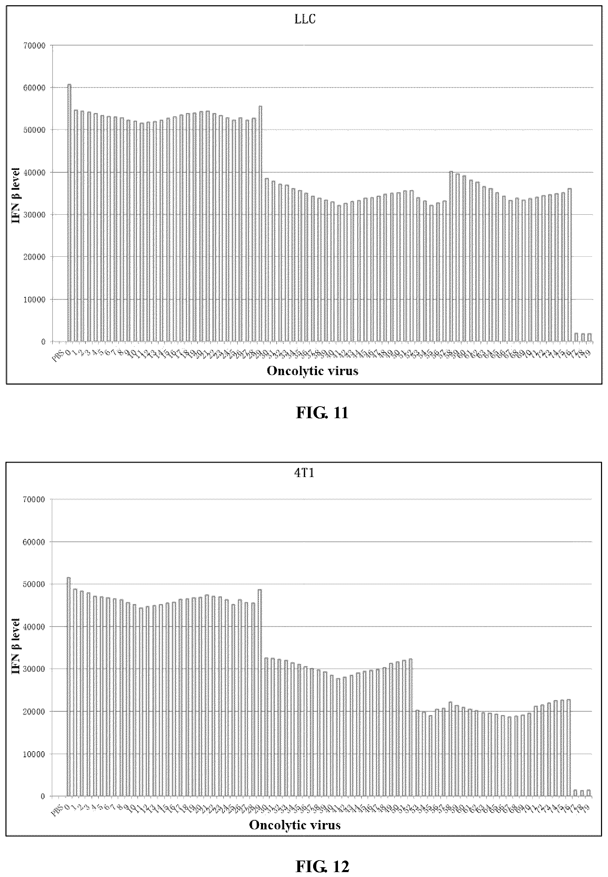

The test results are shown in FIGS. 11-15. As shown in the figures, the number 0 on the horizontal axis represents the wild-type oncolytic virus; the numbers 1-79 on the horizontal axis represent the oncolytic viruses prepared in Preparation Examples 1-79, respectively; the IFN-β level on the vertical axis represents the expression of the IFN-β gene. The larger the value of the IFN-β level, the weaker the reproduction ability of an oncolytic virus in the cell and the easier it is to clear the oncolytic virus; the smaller the value of the IFN-β level, the stronger the reproduction ability of the oncolytic virus in the cell, and the more difficult it is to clear the oncolytic virus.

FIG. 11 shows the expression of IFN-β induced by the oncolytic viruses of the present application and the wild-type oncolytic virus in the LLC cell.

FIG. 12 shows the expression of IFN-β induced by the oncolytic viruses of the present application and the wild-type oncolytic virus in the 4T1 cell.

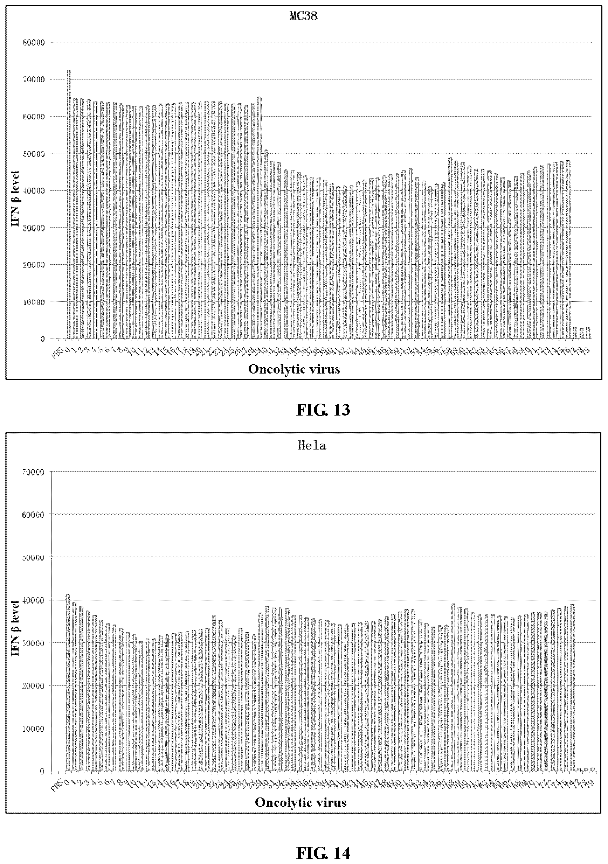

FIG. 13 shows the expression of IFN-β induced by the oncolytic viruses of the present application and the wild-type oncolytic virus in the MC38 cell.

FIG. 14 shows the expression of IFN-β induced by the oncolytic viruses of the present application and the wild-type oncolytic virus in the Hela cell.

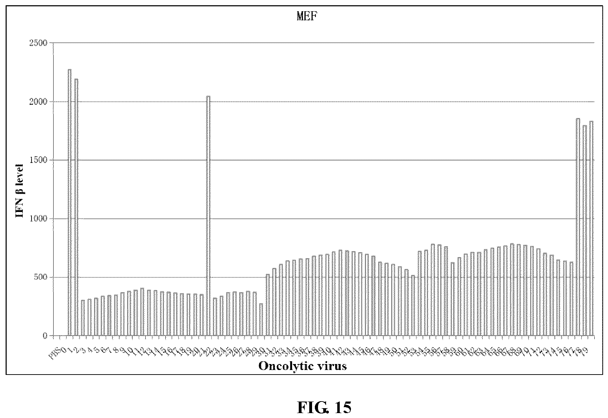

FIG. 15 shows the expression of IFN-β induced by the oncolytic virus of the present application and the wild-type oncolytic virus in the MEF cell.

It can be seen from the above figures that the oncolytic viruses provided in Preparation Examples 1 and 21 of the present application and the wild-type oncolytic virus have weak reproduction ability in the LLC cell, the 4T1 cell, the MC38 cell, and the Hela cell and are all easily cleared. Relatively speaking, the oncolytic viruses provided in Preparation Examples 2-20 and 22-79 are difficultly cleared from the LLC cell, the 4T1 cell, the MC38 cell, and the Hela cell. Especially the oncolytic viruses provided in Preparation Examples 77-79 are more difficultly cleared from the LLC cell, the 4T1 cell, the MC38 cell, and the Hela cell, thereby further ensuring that these oncolytic viruses can better exert their ability to infect and kill the LLC cell, the 4T1 cell, the MC38 cell, and the Hela cell. Moreover, the oncolytic viruses of the present application are more easily cleared from the MEF cell, thereby further ensuring the safety of the MEF cell and improving the safety of the oncolytic viruses.

The specific examples are merely an explanation of the present application and not intended to limit the present application. Those skilled in the art can make modifications, without creative contribution, to the examples as needed after reading this description. Any of the modifications made within the scope of the claims of the present application shall be protected by the Patent Law.

## Claims

1. An oncolytic virus, **characterized by** comprising an M protein, wherein the M protein comprises amino acid substitution(s) at one or more of the following positions compared to an amino acid sequence set forth in SEQ ID NO 1: position 32, position 33, position 49, position 54, position 133, and position 225.

2. The oncolytic virus according to claim 1, **characterized in that**, the amino acid substitution of the M protein comprises mutation of asparagine at position 32 to serine (N32S); and/or mutation of methionine at position 33 to alanine (M33A); and/or mutation of asparagine at position 49 to aspartic acid (N49D); and/or mutation of histidine at position 54 to tyrosine (H54Y); and/or mutation of alanine at position 133 to threonine (A133T); and/or mutation of valine at position 225 to isoleucine (V225I).

3. The oncolytic virus according to claim 2, **characterized in that**, the M protein further comprises amino acid substitution(s) at one or more of the following positions:
position 21, position 51, position 111, position 221, and position 226.

4. The oncolytic virus according to claim 3, **characterized in that**, the amino acid substitution of the M protein further comprises mutation of glycine at position 21 to glutamic acid (G21E); and/or, the amino acid substitution of the M protein comprises mutation of methionine at position 51 to arginine (M51R); and/or, mutation of methionine at position 51 to alanine (M51A); and/or, mutation of leucine at position 111 to alanine (L111A); and/or, mutation of valine at position 221 to phenylalanine (V221F); and/or, mutation of serine at position 226 to arginine (S226R).

5. The oncolytic virus according to any one of claims 1-4, **characterized in that**, the amino acid substitution of the M protein comprises amino acid substitution(s) in any one of the following groups consisting of:
1) G21E and N32S;
2) G21E, N32S, and M33A;
3) G21E, N32S, M33A, and N49D;
4) G21E, N32S, M33A, N49D, and H54Y;
5) G21E, N32S, M33A, N49D, H54Y, and L111A;
6) G21E, N32S, M33A, N49D, H54Y, L111A, and A133T;
7) G21E, N32S, M33A, N49D, H54Y, L111A, A133T, and V225I;
8) G21E, N32S, M33A, N49D, M51R, H54Y, L111A, A133T, and V225I;
9) G21E, N32S, M33A, N49D, M51R, H54Y, L111A, A133T, V221F, and V225I;
10) G21E, N32S, M33A, N49D, M51R, H54Y, L111A, A133T, V221F, V225I, and S226R;
11) N32S, M33A, N49D, M51R, H54Y, L111A, A133T, V221F, V225I, and S226R;
12) M33A, N49D, M51R, H54Y, L111A, A133T, V221F, V225I, and S226R;
13) N49D, M51R, H54Y, L111A, A133T, V221F, V225I, and S226R;
14) M51R, H54Y, L111A, A133T, V221F, V225I, and S226R;
15) H54Y, L111A, A133T, V221F, V225I, and S226R;
16) L111A, A133T, V221F, V225I, and S226R;
17) A133T, V221F, V225I, and S226R;
18) V221F, V225I, and S226R;
19) V225I and S226R;
20) S226R;
21) N32S, N49D, H54Y, and V225I;
22) N32S, N49D, H54Y, V225I, and S226G;
23) N32S, N49D, M51R, H54Y, V221F, V225I, and S226R;
24) N32S, M33A, N49D, M51R, H54Y, V221F, V225I, and S226R;
25) N32S, N49D, M51R, H54Y, A133T, V221F, V225I, and S226R;
26) N32S, M33A, N49D, M51R, H54Y, A133T, V221F, V225I, and S226R; and
27) G21E, N32S, N49D, M51A, H54Y, L111A, V225I, and S226R.

6. The oncolytic virus according to claim 5, **characterized in that**, the amino acid substitution of the M protein comprises G21E, N32S, M33A, N49D, M51R, H54Y, L111A, A133T, V221F, V225I, and S226R.

7. The oncolytic virus according to claim 5, **characterized in that**, the M portion comprises an amino acid sequence as set forth in SEQ ID NO 12.

8. An oncolytic virus, **characterized by** comprising the M protein according to any one of claims 1-7 and further comprising a G protein, wherein the G protein comprises amino acid substitution(s) at one or more of the following positions compared to an amino acid sequence set forth in SEQ ID NO 31: position 438, position 453, position 471, and position 487.

9. The oncolytic virus according to claim 8, **characterized in that**, the G protein further comprises an amino acid substitution(s) at one or more of the following positions: position 53, position 141, position 172, position 217, position 232, position 331, position 371, and position 436.

10. The oncolytic virus according to claim 8 or 9, **characterized in that**, the amino acid substitution of the G protein comprises mutation of valine at position 53 to isoleucine (V53I); and/or mutation of alanine at position 141 to valine (A141V); and/or mutation of aspartic acid at position 172 to tyrosine (D172Y); and/or mutation of lysine at position 217 to glutamic acid (K217E); and/or mutation of aspartic acid at position 232 to glycine (D232G); and/or mutation of valine at position 331 to alanine (V331A); and/or, mutation of valine at position 371 to glutamic acid (V371E); and/or, mutation of glycine at position 436 to aspartic acid (G436D); and/or, mutation of threonine at position 438 to serine (T438S); and/or, mutation of phenylalanine at position 453 to leucine (F453L); and/or, mutation of threonine at position 471 to isoleucine (T471I); and/or, mutation of tyrosine at position 487 to histidine (Y487H).

11. The oncolytic virus according to claim 10, **characterized in that**, the amino acid substitution of the G protein comprises amino acid substitution(s) in any one of the following groups consisting of:
1) V53I;
2) V53I and A141V;
3) V53I, A141V, and D172Y;
4) V53I, A141V, D172Y, and K217E;
5) V53I, A141V, D172Y, K217E, and D232G;
6) V53I, A141V, D172Y, K217E, D232G, and V331A;
7) V53I, A141V, D172Y, K217E, D232G, V331A, and V371E;
8) V53I, A141V, D172Y, K217E, D232G, V331A, V371E, and G436D;
9) V53I, A141V, D172Y, K217E, D232G, V331A, V371E, G436D, and T438S;
10) V53I, A141V, D172Y, K217E, D232G, V331A, V371E, G436D, T438S, and F453L;
11) V53I, A141V, D172Y, K217E, D232G, V331A, V371E, G436D, T438S, F453L, and T471I;
12) V53I, A141V, D172Y, K217E, D232G, V331A, V371E, G436D, T438S, F453L, T471I, and Y487H;
13) A141V, D172Y, K217E, D232G, V331A, V371E, G436D, T438S, F453L, T471I, and Y487H;
14) D172Y, K217E, D232G, V331A, V371E, G436D, T438S, F453L, T471I, and Y487H;
15) K217E, D232G, V331A, V371E, G436D, T438S, F453L, T471I, and Y487H;
16) D232G, V331A, V371E, G436D, T438S, F453L, T471I, and Y487H;
17) V331A, V371E, G436D, T438S, F453L, T471I, and Y487H;
18) V371E, G436D, T438S, F453L, T471I, and Y487H;
19) G436D, T438S, F453L, T471I, and Y487H;
20) T438S, F453L, T471I, and Y487H;
21) F453L, T471I, and Y487H;
22) T471I and Y487H; and
23) Y487H.

12. The oncolytic virus according to claim 11, **characterized in that**, the amino acid substitution of the G protein comprises V53I, A141V, D172Y, K217E, D232G, V331A, V371E, G436D, T438S, F453L, T471I, and Y487H.

13. The oncolytic virus according to claim 11, **characterized in that**, the G protein comprises an amino acid sequence as set forth in SEQ ID NO 43.

14. An oncolytic virus, **characterized by** comprising the M protein according to any one of claims 1-7 or comprising the M protein and G protein according to any one of claims 8-13, and further comprising an N protein, wherein the N protein comprises amino acid substitution(s) at one or more of the following positions compared to an amino acid sequence set forth in SEQ ID NO 55: position 14, position 155, and position 353.

15. The oncolytic virus according to claim 14, **characterized in that**, the amino acid substitution of the N protein comprises mutation of isoleucine at position 14 to valine (I14V); and/or, mutation of arginine at position 155 to lysine (R155K); and/or, mutation of serine at position 353 to asparagine (S353N).

16. The oncolytic virus according to claim 14 or 15, **characterized in that**, the amino acid substitution of the N protein comprises amino acid substitution(s) in any one of the following groups consisting of:
1) I14V;
2) I14V and R155K;
3) I14V, R155K, and S353N;
4) R155K and S353N; and
5) S353N.

17. The oncolytic virus according to claim 16, **characterized in that**, the amino acid substitution of the N protein comprises I14V, R155K and S353N.

18. The oncolytic virus according to claim 16, **characterized in that**, the N portion comprises an amino acid sequence as set forth in SEQ ID NO 58.

19. An oncolytic virus, **characterized by** comprising the M protein according to any one of claims 1-7, or comprising the M protein and G protein according to any one of claims 8-13, or comprising the M protein, G protein and N protein according to any one of claims 14-18, and further comprising a P protein, wherein the P protein comprises amino acid substitution(s) at one or more of the following positions compared to an amino acid sequence set forth in SEQ ID NO 61: position 50, position 76, position 99, position 126, position 140, position 151, position 168, position 170, position 189, and position 237.

20. The oncolytic virus according to claim 19, **characterized in that**, the amino acid substitution of the P protein comprises mutation of arginine at position 50 to lysine (R50K); and/or mutation of valine at position 76 to alanine (V76A); and/or mutation of asparagine at position 99 to glutamic acid (D99E); and/or mutation of leucine at position 126 to serine (L126S); and/or mutation of leucine at position 140 to serine (L140S); and/or mutation of histidine at position 151 to tyrosine (H151Y); and/or mutation of isoleucine at position 168 to methionine (I168M); and/or mutation of lysine at position 170 to glutamic acid (K170E); and/or mutation of tyrosine at position 189 to serine (Y189S); and/or mutation of asparagine at position 237 to aspartic acid (N237D).

21. The oncolytic virus according to claim 19 or 20, **characterized in that**, the amino acid substitution of the P protein comprises amino acid substitution(s) in any one of the following groups consisting of:
1) R50K;
2) R50K and V76A;
3) R50K, V76A and D99E;
4) R50K, V76A, D99E, and L126S;
5) R50K, V76A, D99E, L126S, and L140S;
6) R50K, V76A, D99E, L126S, L140S, and H151Y;
7) R50K, V76A, D99E, L126S, L140S, H151Y, and I168M;
8) R50K, V76A, D99E, L126S, L140S, H151Y, I168M, and K170E;
9) R50K, V76A, D99E, L126S, L140S, H151Y, I168M, K170E, and Y189S;
10) R50K, V76A, D99E, L126S, L140S, H151Y, I168M, K170E, Y189S, and N237D;
11) V76A, D99E, L126S, L140S, H151Y, I168M, K170E, Y189S, and N237D;
12) D99E, L126S, L140S, H151Y, I168M, K170E, Y189S, and N237D;
13) L126S, L140S, H151Y, I168M, K170E, Y189S, and N237D;
14) L140S, H151Y, I168M, K170E, Y189S, and N237D;
15) H151Y, I168M, K170E, Y189S, and N237D;
16) I168M, K170E, Y189S, and N237D;
17) K170E, Y189S, and N237D;
18) Y189S and N237D; and
19) N237D.

22. The oncolytic virus according to claim 21, **characterized in that**, the amino acid substitution of the P protein comprises R50K, V76A, D99E, L126S, L140S, H151Y, I168M, K170E, Y189S, and N237D.

23. The oncolytic virus according to claim 21, **characterized in that**, the P protein comprises an amino acid sequence as set forth in SEQ ID NO 71.

24. An oncolytic virus, **characterized by** comprising the M protein according to any one of claims 1-7, or comprising the M protein and G protein according to any one of claims 8-13, or comprising the M protein, G protein and N protein according to any one of claims 14-18, or comprising the M protein, G protein, N protein, and P protein according to any one of claims 19-23, and further comprising an L protein, wherein the L protein comprises amino acid substitution(s) at one or more of the following positions compared to an amino acid sequence set forth in SEQ ID NO 81:
position 87 and position 487.

25. The oncolytic virus according to claim 24, **characterized in that**, the amino acid substitution of the L protein comprises mutation of serine at position 87 to proline (S87P); and/or mutation of isoleucine at position 487 to threonine (I487T).

26. The oncolytic virus according to claim 24 or 25, **characterized in that**, the amino acid substitution of the L protein comprises amino acid substitution(s) in any one of the following groups consisting of:
1) S87P;
2) S87P and I487T; and
3) I487T.

27. The oncolytic virus according to claim 26, **characterized in that**, the amino acid substitution of the L protein comprises S87P and I487T.

28. The oncolytic virus according to claim 26, **characterized in that**, the L protein comprises an amino acid sequence as set forth in SEQ ID NO 83.

29. The oncolytic virus according to any one of claims 1-28, **characterized by** comprising a rod-shaped virus.

30. The oncolytic virus according to any one of claims 1-29, **characterized by** comprising vesicular stomatitis virus.

31. The oncolytic virus according to any one of claims 1-30, **characterized by** comprising an *Indiana MuddSummer* subtype strain of the vesicular stomatitis virus.

32. The oncolytic virus according to any one of claims 1-31, **characterized by** comprising or expressing a foreign target protein.

33. The oncolytic virus according to any one of claims 1-32, **characterized by** comprising a nucleic acid molecule, wherein the nucleic acid molecule comprises a nucleic acid sequence encoding the M protein with the amino acid substitution(s), and/or a nucleic acid sequence encoding the G protein with the amino acid substitution(s), and/or a nucleic acid sequence encoding the N protein with the amino acid substitution(s), and/or a nucleic acid sequence encoding the P protein with the amino acid substitution(s), and/or a nucleic acid sequence encoding the L protein with the amino acid substitution(s).

34. The oncolytic virus according to claim 33, **characterized in that**, the nucleic acid molecule comprises a nucleic acid sequence encoding the foreign target protein.

35. The oncolytic virus according to claim 34, **characterized in that**, in the nucleic acid molecule, the nucleic acid sequence encoding the foreign target protein is positioned between the nucleic acid sequence encoding the M protein with the amino acid substitution(s), and/or the nucleic acid sequence encoding the G protein with the amino acid substitution(s), and/or the nucleic acid sequence encoding the N protein with the amino acid substitution(s), and/or the nucleic acid sequence encoding the P protein with the amino acid substitution(s), and/or the nucleic acid sequence encoding the L protein with the amino acid substitution(s).

36. An oncolytic virus expression vector, **characterized by** being capable of expressing the oncolytic virus according to any one of claims 1-35.

37. A virus production cell, **characterized by** being capable of producing the oncolytic virus according to any one of claims 1-35.

38. A pharmaceutical composition, **characterized by** comprising the oncolytic virus according to any one of claims 1-35 and, optionally, a pharmaceutically acceptable carrier.

39. A method for preparing the oncolytic virus according to any one of claims 1-35, the oncolytic virus expression vector according to claim 36, the virus production cell according to claim 37, and/or the pharmaceutical composition according to claim 38.

40. A use of the oncolytic virus according to any one of claims 1-35, the oncolytic virus expression vector according to claim 36, the virus production cell according to claim 37, and/or the pharmaceutical composition according to claim 38 in the preparation of a drug for prevention and/or treatment of a disease and/or disorder.

41. The use according to claim 40, **characterized in that**, the oncolytic virus, the oncolytic virus expression vector, the virus production cell and/or the pharmaceutical composition are used in a method for constantly killing an abnormally proliferative cell.

42. The use according to claim 41, **characterized in that**, the abnormally proliferative cell is selected from a tumor cell and a cell related to tumor tissues.

43. The use according to claim 42, **characterized in that**, the tumor comprises a solid tumor or a hematological tumor.
